(19) Europäisches Patentamt
European Patent Office
Office européen des brevets

(11) **EP 2 405 262 B1**

(12) **EUROPEAN PATENT SPECIFICATION**

(45) Date of publication and mention of the grant of the patent:
**28.10.2020 Bulletin 2020/44**

(51) Int Cl.:
***G01N 25/04*** *(2006.01)* ***C12Q 1/68*** *(2018.01)*
***G01N 33/53*** *(2006.01)*

(21) Application number: **11173124.6**

(22) Date of filing: **07.07.2011**

(54) **Nucleic acid abundance ratio measurement device, method, and program storage medium, determination method and use of a nucleic acid abundance ratio measurement kit**

Verhältnismessvorrichtung der Nukleinsäureabundanz, Verfahren, Programmspeichermedium, Bestimmungsverfahren und Verwendung eines Kits zur Messung des Verhältnisses der Nukleinsäureabundanz

Dispositif de mesure du taux d'abondance d'acides nucléiques, procédé et support de stockage de programmes, procédé de détermination et l'utilization d'un kit de mesure du taux d'abondance d'acides nucléiques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.07.2010 JP 2010155032**
**04.07.2011 JP 2011148520**

(43) Date of publication of application:
**11.01.2012 Bulletin 2012/02**

(73) Proprietor: **ARKRAY, Inc.**
**Minami-ku**
**Kyoto-shi**
**Kyoto 601-8045 (JP)**

(72) Inventors:
- **Hirano, Katsuyasu**
  **Kyoto, 601-8045 (JP)**
- **Komori, Mariko**
  **Kyoto, 601-8045 (JP)**

(74) Representative: **Dehns**
**St. Bride's House**
**10 Salisbury Square**
**London EC4Y 8JD (GB)**

(56) References cited:
**WO-A1-2009/081965** **WO-A2-2005/026377**
**JP-A- 2007 143 420** **US-A1- 2007 128 608**
**US-A1- 2007 224 598** **US-A1- 2008 176 226**
**US-A1- 2009 155 791**

- **WILHELM ET AL: "SoFAR: Software for Fully Automatic Evaluation of Real-Time PCR Data", BIOTECHNIQUES, INFORMA HEALTHCARE, US, vol. 34, no. 2, 1 January 2003 (2003-01-01), pages 324-332, XP003024150, ISSN: 0736-6205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 405 262 B1

SEQUENCE LISTING

<110> ARKRAY, INC.

<120> Nucleic Acid Abundance Ratio Measurement Device, Method and Program Storage Medium, Determination Method, and Nucleic Acid Abundance Ratio Measurement Kit

<130> CO-F03565-00

<150> JP2010-155032
<151> 2010-07-07

<150> JP2011-148520
<151> 2011-07-04

<160> 5

<170> PatentIn version 3.4

<210> 1
<211> 21

<212> DNA
<213> Artificial

<220>
<223> primer_F

<400> 1 tgtattcaca gagacttggc a 21

<210> 2
<211> 21
<212> DNA
<213> Artificial

<220>
<223> primer_R

<400> 2 gagaatgggt actcacgttt c 21

<210> 3
<211> 18
<212> DNA
<213> Artificial

<220>
<223> prove

<400> 3 gatgtctctg gctagacc 18

<210> 4
<211> 300
<212> DNA
<213> Artificial

<220>
<223> Wt

<400> 4

cactatagta ttaaaaagtt agttttcact ctttacaagt taaaatgaat ttaaatggtt 60

ttcttttctc ctccaaccta atagtgtatt cacagagact tggcagccag aaatatcctc 120

cttactcatg gtcggatcac aaagatttgt gattttggtc tagccagaga catcaagaat 180

gattctaatt atgtggttaa aggaaacgtg agtacccatt ctctgcttga cagtcctgca 240

aaggattttt agtttcaact ttcgataaaa attgtttcct gtgactttca taatgtaaat 300

<210> 5
<211> 300
<212> DNA

<213> Artificial

<220>
<223> Mt

<400> 5

cactatagta ttaaaaagtt agttttcact ctttacaagt taaaatgaat ttaaatggtt 60

ttcttttctc ctccaaccta atagtgtatt cacagagact tggcagccag aaatatcctc 120

cttactcatg gtcggatcac aaagatttgt gattttggtc tagccagagt catcaagaat 180

gattctaatt atgtggttaa aggaaacgtg agtacccatt ctctgcttga cagtcctgca 240

aaggattttt agtttcaact ttcgataaaa attgtttcct gtgactttca taatgtaaat 300

## Description

BACKGROUND

Field of the Invention

The present invention relates to a nucleic acid abundance ratio measurement device, a nucleic acid abundance ratio measurement method, a nucleic acid abundance ratio measurement program storage medium, a determination method and a use of a nucleic acid abundance ratio measurement kit.

A proposal has been made for a melting curve analysis method and a melting curve analyzing device for discriminating the type of a polymorphous gene by determining the presence or absence of two different peaks and their temperature ranges from a melting curve expressed by a differential melting curve that expresses a relationship between a signal differential value, which is a differential of a signal value of a melting curve expressing signal values of a sample in a range of temperatures, and temperature (see, for example, International Publication (WO) No. 2009/081965).

However, while the proposal of WO 2009/081965 is capable of automatically discriminating the type of a polymorphous gene, it is not capable of obtaining the proportion of the gene that is mutated. Related prior art in this field includes US 2007/224598 which discloses a system and methods for identifying JAK2-specific polynucleotide sequences in a biological sample including oligonucleotide primer and probe compositions for detecting mutations in JAK2 polynucleotides, and specifically the $JAK2^{V617F}$ mutation. US 2009/155791 discloses a method for detecting the methylation status of CpG-containing nucleic acids by nucleic acid amplification and melting curve analysis of amplification products. Wilhelm et al. ("SoFAR: Software for Fully Automatic Evaluation of Real-Time PCR Data", Biotechniques, Informa Healthcare, 2003, 34:2, pages 324-332) discloses software which automatically evaluates the data acquired with the LightCycler™ system and applies algorithms for an adaptive background correction of signal trends, the calculation of the effective signal noise, the automated identification of the exponential phases, the adaptive smoothing of the raw data, and the correction of melting curve data. JP 2007 143420 A and US 2007/128608 A1 disclose further methods for determining an amount of a nucleic acid in a mixture.

SUMMARY

In consideration of the above circumstances, the present invention provides a nucleic acid abundance ratio measurement device, a nucleic acid abundance ratio measurement method, a nucleic acid abundance ratio measurement program storage medium, a determination method and a use of a nucleic acid abundance ratio measurement kit that are capable of measuring a nucleic acid abundance ratio by easily computing a nucleic acid abundance ratio.

A first aspect of the present invention is a nucleic acid abundance ratio measurement device according to claim 1 including: a detection section that detects a detection signal over different temperature ranges of a melting curve for a nucleic acid mixture having one or more melting temperatures; and an abundance ratio computation section that computes a nucleic acid abundance ratio based on a ratio of characteristic amounts obtained from the detection signal detected by the detection section and based on detection amount data. The detection amount data is data expressing a relationship between ratios of characteristic amounts and nucleic acid abundance ratios, and is, for example a detection amount curve, table, or computation formula expressing the relationship between the ratios of characteristic amounts and the nucleic acid abundance ratios.

According to the first aspect, the detection section detects a detection signal over different temperature ranges of a melting curve for a nucleic acid mixture having one or more melting temperatures. Then the abundance ratio computation section measures a nucleic acid abundance ratio in a sample (a nucleic acid mixture to be measured) by computing a nucleic acid abundance ratio based on a ratio of characteristic amounts obtained from the detection signal detected by the detection section and the detection amount data.

The first aspect may further include: a storage section that stores the detection amount data that, based on respective melting curves each expressing a relationship between temperature and a detection signal obtained from each of a plurality of nucleic acid mixtures of different abundance ratios of nucleic acids having different melting temperatures, expresses a relationship between ratios of characteristic amounts obtained from the detection signal of the melting curves over a plurality of temperature ranges respectively containing the melting temperatures, and the abundance ratios; or an input section that inputs the detection amount data.

According to this configuration, the storage section stores detection amount data that, based on respective melting curves each expressing the relationship between temperature and detection signal obtained from each of plural nucleic acid mixtures of different abundance ratios of nucleic acids having different respective melting temperatures, expresses a relationship between ratios of characteristic amounts obtained from the detection signal of the melting curves over plural temperature ranges respectively containing the melting temperatures, and the abundance ratios. Or the detection amount data is input via the input section. The nucleic acid abundance ratio of a sample is measured by computing the nucleic acid abundance ratio based on the detection amount data and the ratio of characteristic amounts, which is computed based on the detection signals over the respective plural temperature ranges of the melting curve of the nucleic acid mixture to be measured (measurement target).

Accordingly, the nucleic acid abundance ratio in a sample can be measured by simple computation based on the detection amount data expressing the relationship between plural abundance ratios and the ratio of characteristic amounts obtained from the detection signal of the melting curves over the plural temperature ranges containing the melting temperatures of the respective nucleic acids, and the ratio of characteristic amounts obtained from the detection signal of the melting curve over the plural temperature ranges of the measurement target nucleic acid mixture.

The characteristic amount ratio is set as a ratio, in a differential melting curve expressing a relationship between temperature and differential values of the detection signal, of a first surface area of a region bounded by a straight line passing through a point of the differential melting curve corresponding to a lower limit value of a first temperature range and a point corresponding to an upper limit value of the first temperature range and bounded by the melting curve, and a second surface area of a region bounded by a straight line passing through a point corresponding to a lower limit value of a second temperature range and a point corresponding to an upper limit value of the second temperature range and bounded by the melting curve.

The lower limit values or the upper limit values of the first and second temperature ranges can be selected from: temperatures at which the differential value takes a minimum value between two peaks of the second melting curve, temperatures corresponding to tails of the peak of the second melting curve, or temperatures within ±15°C from a temperature corresponding to the peak of the second melting curve.

Or, the lower limit values or the upper limit values of the first and second temperature ranges may be temperatures within ±10°C from a temperature corresponding to the peak of the second melting curve.

Further alternatively, the lower limit values or the upper limit values of the first and second temperature ranges may be temperatures within ±7°C from a temperature corresponding to the peak of the second melting curve.

In these configurations, the upper limit value of one of the first or second temperature range may be different from the lower limit value of the other of the first or second temperature range that is a higher range than that of the one of the first or second temperature range.

A width from the lower limit value to the upper limit value of the first temperature range and a width from the lower limit value to the upper limit value of the second temperature range may be identical.

Or, a width from the lower limit value to the upper limit value of the first temperature range and a width from the lower limit value to the upper limit value of the second temperature range may be different.

The first aspect may further include: a temperature controller that controls change in temperature of the nucleic acid mixture; and a measurement section that measures degree of light absorption, fluorescence intensity or relative fluorescence intensity as the detection signal. By adopting such a configuration the measured detection signal from a sample can be employed to determine the nucleic acid abundance ratio in the sample.

A second aspect of the present invention is a nucleic acid abundance ratio measurement method according to claim 9 including: detecting a detection signal over different temperature ranges for a melting curve of a nucleic acid mixture having one or more melting temperatures; and computing a nucleic acid abundance ratio based on a ratio of characteristic amounts obtained from the detection signal that has been detected and based on detection amount data.

The above nucleic acid abundance ratio measurement method detects a detection signal over different temperature ranges for a melting curve of a nucleic acid mixture having one or more melting temperatures. Then a nucleic acid abundance ratio in a sample is measured by computing the nucleic acid abundance ratio based on a ratio of characteristic amounts obtained from the detection signal that has been detected and based on detection amount data.

A third aspect of the present invention is a nucleic acid abundance ratio measurement method including: generating detection amount data based on melting curves each expressing a relationship between temperature and a detection signal obtained from each of a plurality of nucleic acid mixtures of different abundance ratios of nucleic acids having different respective melting temperatures, the detection amount data expressing a relationship between ratios of characteristic amounts obtained from the detection signal of the melting curves over a plurality of temperature ranges respectively containing the melting temperatures, and the abundance ratios; computing a ratio of characteristic amounts based on a detection signal of a melting curve of a measurement target nucleic acid mixture over temperature ranges corresponding to each of the plurality of respective temperature ranges; and computing a nucleic acid abundance ratio based on the computed ratio of characteristic amounts and the generated detection amount data. The nucleic acid abundance ratio can be measured by computing the nucleic acid abundance ratio through these processes

A fourth aspect of the present invention is a non-transitory storage medium according to claim 11, storing a program that causes a computer to execute abundance ratio measurement processing, the abundance ratio measurement processing including: receiving a detection signal of a melting curve of a nucleic acid mixture having one or more melting temperatures, the detection signal being detected over different temperature ranges; and computing a nucleic acid abundance ratio based on a ratio of characteristic amounts obtained from the received detection signal and based on detection amount data.

A fifth aspect of the present invention is a non-transitory storage medium storing a program that causes a computer to execute abundance ratio measurement processing, the abundance ratio measurement processing including: acquiring detection amount data from a storage section that stores the detection amount data that, based on respective melting curves each expressing a relationship between temperature and a detection signal obtained for each of a plurality of nucleic acid mixtures of different abundance ratios of nucleic acids having different respective melting temperatures, expresses a relationship between ratios of characteristic amounts obtained from the detection signals of the melting curves over a plurality of temperature ranges respectively containing the melting temperatures, and respective abundance ratios, or acquiring the detection amount data input by an input section; computing a ratio of characteristic amounts based on a detection signal of a melting curve of a measurement target nucleic acid mixture, the detection signal detected over temperature ranges respectively corresponding to each of the plurality of temperature ranges; and computing a nucleic acid abundance ratio based on the computed ratio of characteristic amounts and the acquired detection amount data. A computer is accordingly able to measure the abundance ratio of nucleic acids in a test sample. An alternative aspect is a nucleic acid abundance ratio measurement program that causes the computer to function as a controller that controls another computer to perform computation of the detection signal or a portion of the computation.

A sixth aspect of the present invention is a determination method according to claim 13 of determining a condition of a patient based on: a nucleic acid abundance ratio obtained with any one of the preceding aspects; and a relationship determined in advance between nucleic acid abundance ratios and patient condition.

A seventh aspect of the present invention is a determination method according to claim 14 of determining a constitution of a patient and/or an appropriate drug administration amount for the constitution based on: a nucleic acid abundance ratio obtained with any one of the preceding aspects; and a relationship determined in advance between nucleic acid abundance ratios and constitutions and/or appropriate drug administration amount for the constitutions.

An eighth aspect of the present invention is a use of a nucleic acid abundance ratio measurement kit according to claim 15 in the measurement of a nucleic acid abundance ratio with any one of the preceding aspects, the kit including: a probe that can be hybridized with a region of a nucleic acid sequence that includes a target mutation that may exist in the nucleic acid mixture; and a set of primers that can amplify the nucleic acid sequence that includes the target mutation. The nucleic acid abundance ratio measurement kit can be also used for determining a constitution and/or an appropriate drug administration amount for the constitution based on the relationship between the predetermined nucleic acid abundance ratio and the constitution and/or the appropriate drug administration amount for the constitution.

Another aspect of the present invention is a nucleic acid abundance ratio measurement device that includes: a storage section that stores detection amount data that, based on respective melting curves expressing the relationship between temperature and detection signal obtained from each of plural nucleic acid mixtures of different abundance ratios of nucleic acids in plural respective abundance ratios, expresses a relationship between ratios of characteristic amounts obtained from the detection signal of the melting curves over plural temperature ranges containing respective different melting temperatures and the abundance ratios; a characteristic amount ratio computation section that computes a ratio of characteristic amounts based on a detection signal of a melting curve of a measurement target nucleic acid mixture over temperature ranges corresponding to the plural respective temperature ranges; and a nucleic acid abundance ratio computation section that computes a nucleic acid abundance ratio based on the ratio characteristic amounts computed by the characteristic amount ratio computation section and the detection amount data stored in the storage section.

According to the above aspects for measuring abundance ratio of nucleic acids as explained above, an abundance ratio of nucleic acids in a sample can be measured easily by computing the nucleic acid abundance ratio based on a ratio of characteristic amounts obtained from a detection signal of a melting curve of a mixture of nucleic acids having different melting temperatures and based on detection amount data.

According to the aspects of determination method and kit, the nucleic acid abundance ratio obtained by any one of the aspects for measuring abundance ratio of nucleic acids can be employed to determine the condition of a patient.

BRIEF DESCRIPTION OF THE DRAWINGS

Exemplary embodiments of the present invention will be described in detail based on the following figures, wherein:

Fig. 1 is a block diagram illustrating a configuration of a nucleic acid abundance ratio determination device as described herein;
Fig. 2A is a graph illustrating an example of a melting curve of a nucleic acid mixture, and Fig. 2B is a graph illustrating a differential melting curve of the example;
Fig. 3 is a graph illustrating another example of a differential melting curve;
Fig. 4 is a graph illustrating an example of a detection amount curve;
Fig. 5 is a flow chart illustrating the contents of a nucleic acid abundance ratio measurement routine for a nucleic acid abundance ratio determination device of the exemplary embodiment;
Fig. 6 is a graph illustrating differential melting curves for each sample of nucleic acid mixture in an Example;
Fig. 7 is a graph illustrating a melting curve of a nucleic acid mixture to be measured (measurement target) in the Example;
Fig. 8 is a graph illustrating a differential melting curve of the measurement target nucleic acid mixture in the Example;
Fig. 9A is a graph illustrating another example of a melting curve of a nucleic acid mixture, and Fig. 9B is a graph illustrating a differential melting curve of the another example;
Fig. 10A and Fig. 10B are graphs for explaining another example of a ratio of characteristic amounts;
Fig. 11 is a diagram illustrating an example of a differential of a melting curve in which two temperature ranges are separated from each other; and
Fig. 12 is a graph illustrating an example of a differential melting curve in which there is a significant difference in base values.

DETAILED DESCRIPTION

As shown in Fig. 1, a nucleic acid abundance ratio measurement device 10 as described herein includes: an operation section 12 configured with a keyboard, mouse, touch panel and/or a reader, such as barcode reader, and operated to input various types of data; a display section 14 that displays results of nucleic acid abundance ratio measurement; and a computer 16 that executes nucleic acid abundance ratio measurement processing. A nucleic acid abundance ratio measurement device of the invention comprises an abundance ratio computation section and a detection section.

The computer 16 includes: a CPU 20 that controls the nucleic acid abundance ratio measurement device 10 overall; ROM 22 that serves as a storage medium that stores various programs, including a program for nucleic acid abundance ratio measurement processing as described later; RAM 24 that serves as a working area for temporary storage of data; a memory 26 that serves as a storage section in which various data is stored; an input-output port (I/O port) 28; a network interface (network I/F) 30; and a bus that connects these sections together. The operation section 12 and the display section 14 are connected to the I/O port 28. A HDD may also be provided to the computer 16.

The memory 26 is stored with a detection amount curve that, based on melting curves for plural nucleic acid mixtures having different abundance ratios of two types of nucleic acid, expresses the relationship between, over two temperature ranges that include different melting temperatures, a ratio of the sum of differential values of a detection signal for a melting curve from which the sum of background levels thereof has been subtracted, and the abundance ratio of the two types of nucleic acid. The detection amount curve may be stored in the ROM 22 or in a HDD. In the present exemplary description, explanation is given of a case in which stored detection amount curves are read out prior to computation. However configuration may be made such that detection amount curve data is input to the operation section 12 by, for example, reading in a bar code including data for the detection amount curves with a bar code reader, reading in the detection amount curve data stored on an IC chip or RFID with various readers, or receiving the detection amount curve from an external device connected through the I/O port 28 and the network I/F 30.

Explanation follows regarding a detection amount curve generation method.

First, for example, plural nucleic acid mixtures are prepared that each have different abundance ratios of two types of nucleic acid, a wild-type nucleic acid Wt and a mutant nucleic acid Mt. Melting curves are obtained with a melting curve analysis instrument for each of the plural nucleic acid mixtures.

Fig. 2A illustrates a melting curve expressing the relationship for a single nucleic acid mixture of a detection signal, such as a degree of light absorption or fluorescence intensity, to temperature. Fig. 2B illustrates a melting curve (also called a differential melting curve, which corresponds to the second melting curve of the present invention) expressing the relationship of the differential values of the detection signal to temperature. The melting temperature $Tm_W$ of the nucleic acid Wt and the melting temperature $Tm_M$ of the mutant nucleic acid Mt are detected from the peaks of the differential melting curve, and temperature ranges that contain $Tm_W$ and $Tm_M$, respectively, are set. A temperature range $\Delta T_W$ containing $Tm_M$ can be set, for example, with a lower limit at the temperature at which the differential value of the detection signal reaches a minimum between $Tm_W$ and $Tm_M$, and with an upper limit at the temperature corresponding to the tail of the peak of the detection signal. A temperature range $\Delta T_M$ containing $Tm_M$ can be set, for example, with an upper limit at the temperature at which the differential value of the detection signal reaches a minimum between $Tm_W$ and $Tm_M$, and with a lower limit at a temperature corresponding to the tail of the peak of the detection signal. The temperature range $\Delta T_W$ and the temperature range $\Delta T_M$ can be set so as to have the same width as each other (for example 10°C) as shown in Fig. 2B, or set to have different widths from each other (for example a temperature range $T_W$ of 10°, and a temperature range $T_M$ of 7°C) as shown in Fig. 3. Further, the temperature range $\Delta T_W$ and the temperature range $\Delta T_M$ can be set with widths from minus X°C to plus X°C from the melting temperatures $Tm_W$ and $Tm_M$, respectively, (X may be for example 15°C or less, preferably 10°C or less, and more preferably 7°C or less). Such setting of temperature ranges can be easily implemented automatically.

Then, for each of the temperature range $\Delta T_W$ and the temperature range $\Delta T_M$, a surface area is derived of an area bounded by a straight line passing through a point corresponding to the lower limit and a point corresponding to the upper limit of the respective temperature range of the differential melting curve and bounded by the differential melting curve itself (i.e., the shaded regions in Fig. 2B). A specific example for a method that can be employed for deriving the surface area is set out below. The surface area S can be derived from the following Equation (1), wherein f (T) is a differential value of the detection signal at temperature T, and B (T) is a base value at temperature T.

$$\text{Surface Area } S = \{f(T_{s+1}) - B(T_{s+1})\} + \{f(T_{s+2}) - B(T_{s+2})\} \text{ and so on up to } \{f(T_{e-1}) - B(T_{e-1})\} \qquad \text{Equation (1)}$$

wherein $T_s$ is the lower limit value of each of the temperature ranges, and $T_e$ is the upper limit value thereof. The base value B (T) at each temperature T is a value derived according to the following Equation (2), and represents the background level included in the detection signal. Influence from background included in the detection signal can be appropriately removed by subtracting this base value from the differential value of the detection signal.

$$B(T) = a \times (T - T_s) + f(T_s) \qquad \text{Equation (2)}$$

wherein

$$a = \{f(T_e) - f(T_s)\} / (T_e - T_s).$$

For each nucleic acid mixture the surface area $S_W$ over the temperature range $\Delta T_W$ and the surface area $S_M$ over the temperature range $\Delta T_M$ are derived according to Equation (1) and Equation (2). A detection amount curve is then generated that expresses the relationship between the surface area ratios and the abundance ratios for each of the nucleic acid mixtures. Fig. 4 illustrates an example of a detection amount curve, with the abundance ratio (the proportion of nucleic acid Mt to the total nucleic acid mixture) on the horizontal axis and the surface area ratio ($S_M/S_W$) on the vertical axis. The thus generated detection amount curve is stored in the memory 26. The surface area ratio may also be defined as ($S_W/S_M$).

Explanation follows, with reference to Fig. 5, regarding a nucleic acid abundance ratio measurement routine executed by the nucleic acid presence ratio measurement device 10 described herein on a nucleic acid mixture to be measured (measurement target).

At step 100, the detection amount curve stored in the memory 26 is read in to the device 10.

Then, at step 102, melting curve data is acquired for a subject with unknown abundance ratio of two nucleic acids (the measurement target nucleic acid mixture). The melting curve data can, for example, be acquired from an external device, such as a melting curve analysis instrument connected through the network I/F 30, or be acquired by reading in data stored on a storage medium. Devices of the invention comprise a detection section and an abundance ratio computation section.

At step 104, the detection signal of the melting curve acquired at step 102 is differentiated with respect to temperature, and a differential melting curve is computed that expressed the relationship of the differential value of the detection signal against temperature.

At step 106, temperature ranges $\Delta T'_W$ and $\Delta T'_M$ are set for the differential melting curve computed at step 104, corresponding to the respective temperature ranges $\Delta T_W$ and $\Delta T_M$, which have been set when the detection amount curve read in at step 100 was generated. The temperature ranges $\Delta T'_W$ and $\Delta T'_M$ may be set as the same temperature ranges as the temperature ranges $\Delta T_W$ and $\Delta T_M$, which have been set when the detection amount curve was generated, or may be set as temperature ranges approximating to the temperature ranges $\Delta T_W$ and $\Delta T_M$.

The surface area is then derived in each of the temperature ranges $\Delta T'_W$ and $\Delta T'_M$ for an area bounded by a straight line passing through the point corresponding to the lower limit and the point corresponding to the upper limit of the temperature range of the differential melting curve, and bounded by the differential melting curve itself. Specifically, the surface area $S'_W$ for the temperature range $\Delta T'_W$ and the surface area $S'_M$ for the temperature range $\Delta T'_M$ are computed according to Equation (1) and Equation (2) by a similar method as that employed to computing the surface area of each of the temperature ranges when the detection amount curve was generated.

At step 108, the surface area ratio $S'_M/S'_W$ is computed with the surface areas $S'_W$ and $S'_M$ that have been computed at step 106.

At step 110, based on the surface area ratio $S'_M/S'_W$ computed at step 108 and the detection amount curve read at step 100, the abundance ratio of nucleic acids in the sample is measured by computing the nucleic acid abundance ratio corresponding to the surface area ratio $S'_M/S'_W$. Thereby, the mutation rate in the sample is determined.

Then, at step 112, the measurement result at step 110 is output by displaying the measurement result on the display section 14, and processing is completed.

In the above-described routine, the detection amount curve is read (at step 100) before the melting curve is obtained at step 102. However, embodiments are not limited to this, and the reading of the detection amount curve can be performed at any time before the abundance ratio of nucleic acids (the mutation rate) is measured based on the surface area ratio and the detection amount curve.

As explained above, according to the nucleic acid abundance ratio measurement device as described herein, the abundance ratio of nucleic acids in a sample can be readily determined by simple computation based on: the detection amount curve, expressing the relationship between abundance ratios of two types of nucleic acid, and ratios of surface areas obtained from differential melting curves at two temperature ranges respectively containing the melting temperatures of each nucleic acid; and a surface area ratio obtained from a differential melting curve of a measurement target nucleic acid mixture of unknown abundance ratio at temperature ranges corresponding to the two temperature ranges of the differential melting curve. Thereby, the mutation rate in the sample can be readily determined.

- Example 1

Explanation follows regarding an Example of the present exemplary embodiment in which a c-Kit gene partial sequence is employed as the measurement target nucleic acid.

PCR and Tm analysis is performed on a sample of nucleic acid mixtures (at $10^3$ copies/reaction liquid) as shown in Table 3, with a full automatic SNP analyzer (product name: i-densy®; produced by Arkray Inc.). The composition of the PCR reaction liquid is shown in Table 1 and the conditions of the PCR and Tm analysis is shown in Table 4.

Table 1

| In 50μL of PCR Reaction Liquid Composition: |
|---|
| 1 × reaction buffer |
| 1.25U Taq polymerase |
| 1.5 mmol/L $MgCl_2$ |
| 0.2 mmol/L dNTP |
| 1 μmol/L F-primer * |
| 0.5 μmol/L R-primer* |
| 0.2 μmol/L probe* |
| *Sequences of F-primer, R-primer and the probe are shown in Table 2 (SEQ ID 1-3) |

Table 2

| Name | Sequence | Mer |
|---|---|---|
| F-primer (SEQ ID 1) | 5' -tgtattcacagagacttggca-3' | 21 |
| R-primer (SEQ ID 2) | 5' -gagaatgggtactcacgtttc-3' | 21 |
| Probe (SEQ ID 3) | 5 '-gatagtctctggctagacc-(BODIPY FL)-3' | 18 |

Table 3

| Sample Name | Mixing Proportions of Each Plasmid | |
|---|---|---|
| | Wt | Mt |
| Wt 1 00% | 100% | 0% |
| Mt 10% | 90% | 10% |
| Mt 20% | 80% | 20% |

(continued)

| Sample Name | Mixing Proportions of Each Plasmid | |
|---|---|---|
| | Wt | Mt |
| Mt 30% | 70% | 30% |
| Mt 40% | 60% | 40% |
| Mt 50% | 50% | 50% |
| Mt 60% | 40% | 60% |
| Mt 70% | 30% | 70% |
| Mt 80% | 20% | 80% |
| Mt 90% | 10% | 90% |
| Mt 100% | 0% | 100% |
| Note that in this Example, Wt plasmid = 100% and Mt plasmid = 100% are also regarded as nucleic acid mixtures.. | | |

For the plasmid, pT7 Blue T-vector (trade name, available through TaKaRa Bio Inc.) to which the sequences (Wt Sequence: SEQ ID4, Mt Sequence: SEQ ID5) shown below are inserted into, and linearized using Eco R1, is used. The Wt Sequence and the Mt Sequence differ in the base shown in capitals. The plasmid inserted with the Wt Sequence is referred to as the Wt plasmid, and the plasmid inserted with the Mt Sequence is referred to as the Mt plasmid.

Wt Sequence (SEQ ID 4):

cactatagtattaaaaagttagttttcactctttacaagttaaaatgaatttaaatggttttcttttctcctccaacctaatagtgtattca
cagagacttggcagccagaaatatcctccttactcatggtcggatcacaaagatttgtgattttggtctagccagagAcatcaagaat
gattctaattatgtggttaaaggaaacgtgagtacccattctctgcttgacagtcctgcaaaggatttttagtttcaactttcgataaaa
attgtttcctgtgactttcataatgtaaat

Mt Sequence (SEQ ID 5):

cactatagtattaaaaagttagttttcactctttacaagttaaaatgaatttaaatggttttcttttctcctccaacctaatagtgtattca
cagagacttggcagccagaaatatcctccttactcatggtcggatcacaaagatttgtgattttggtctagccagagTcatcaagaatg
attctaattatgtggttaaaggaaacgtgagtacccattctctgcttgacagtcctgcaaaggatttttagtttcaactttcgataaaaa
ttgtttcctgtgactttcataatgtaaat

Table 4

| Conditions of PCR and Tm Analysis |
|---|
| 95°C 60 sec |
| ↓ |
| (95°C 1sec, 58°C 15 sec) × 50 cycles |
| ↓ |
| 95°C 1sec |
| ↓ |
| 40°C 60sec |
| ↓ |
| melting curve (measure across range 40°C to 75°C at 1°C/3 sec) |

Fig. 6 illustrates differential melting curves of the samples measured according to the above conditions. In the present Example, fluorescence intensity is obtained as the detection signal. The peaks are extracted from the differential melting curves of Fig. 6, and the melting temperatures $Tm_W$ and $Tm_M$ are determined for each of the nucleic acids. The temperature range $\Delta T_W$(58°C to 66°C) and the temperature range $\Delta T_M$ (50°C to 58°C) are then set so that the determined melting temperatures are respectively included therein. Then, the surface area $S_W$ for the temperature range $\Delta T_W$ and the surface area $S_M$ for the temperature range $\Delta T_M$ are derived according to Equation (1) and Equation (2), and the surface area ratio $S_M/S_W$ is calculated. Table 5 illustrates the surface areas $S_M$ and $S_W$ and the surface area ratio $S_M/S_W$ for each of the samples.

Table 5

| Sample Name | $S_M$ | Sw | $S_M/S_W$ |
|---|---|---|---|
| Wt 1 00% | 0 | 879 | 0.0% |
| Mt 10% | 0 | 924 | 0.0% |
| Mt 20% | 39.6 | 800.5 | 4.9% |
| Mt 30% | 121.9 | 652 | 18.7% |
| Mt 40% | 147.8 | 619 | 23.9% |
| Mt 50% | 238.5 | 509 | 46.9% |
| Mt 60% | 333.5 | 433.5 | 76.9% |
| Mt 70% | 375 | 346 | 108.4% |
| Mt 80% | 471 | 192 | 245.3% |
| Mt 90% | 618.5 | 43.5 | 1421.8% |
| Mt 100% | 632.5 | 0 | - |

A detection amount curve is generated based on the values shown in Fig. 5, with the proportion of Mt in the total sample shown on the horizontal axis and the surface area ratio $S_M/S_W$ shown on the vertical axis. The generated detection amount curve here is the same as the example shown in Fig. 4.

This detection amount curve is employed to measure the nucleic acid abundance ratio of a measurement target nucleic acid mixture of unknown Wt and Mt abundance ratio.

First, melting curve data, as illustrated in Fig. 7, expressing the relationship of fluorescence intensity against temperature of the measurement target nucleic acid mixture is acquired (step 102). Then, as shown in Fig. 8, differentials of the fluorescence intensities in the melting curve are taken, and a differential melting curve expressing the relationship of fluorescence intensity differentials against temperature is computed (step 104).

The temperature ranges $\Delta T'_W$ and $\Delta T'_M$ as the same temperature ranges as the temperature range $\Delta T_W$(58°C to 66°C) and the temperature range $\Delta T_M$ (50°C to 58°C), which has been set when generating the detection amount curve of Fig. 4, are set for the computed differential melting curve. The surface area $S'_W$ for the temperature range $\Delta T'_W$ and the surface area $S'_M$ for the temperature range $\Delta T'_M$ are computed according to Equation (1) and Equation (2) (step 106). In this Example, $S'_W = 730$ and $S'_M = 241.5$ are obtained.

The surface area ratio $S'_M/S'_W$ is based on the computed surface areas $S'_W$ and $S'_M$ (step 108). In this Example, $S'_M/S'_W = 0.331$ (33.1%) is obtained.

The mutation rate is determined by computing the proportion of Mt, that is the mutation rate, corresponding to the surface area ratio $S'_M/S'_W$ (= 33.1%) (step 110). In this Example, the proportion of Mt is between 40 to 50%.

Explanation in the above Example is of a case in which a probe that emits fluorescent light is used, and the fluorescence intensity emitted in response to excitation light appropriate for the fluorescent dye is employed as the detection signal indicating the melting state. However, embodiments are not limited to this. For example, the degree of light absorption at 260nm that increases due to dissociation of double-stranded nucleic acid may be employed as the detection signal. Further, explanation in the above Example is of a case in which a probe that quenches when double-strands are formed (not dissociated) is employed; however a probe may be employed that emits fluorescent light when double-strands are formed. In such cases, a melting curve as shown in Fig. 9A is obtained that expresses the relationship of fluorescence intensity against temperature. A differential melting curve such as shown in Fig. 9B is obtained therefrom.

Specific examples of fluorescent dyes include intercalaters such as ethidium bromide and SYBR® Green. Generally, in such fluorescent dyes, fluorescent light is emitted by double-strand formation, and the emission of fluorescent light is suppressed by double-strands dissociating. Fluorescent dyes may, for example, be conjugated to single-strand nucleic acid of at least one of the double-strands configuring the nucleic acid. Examples of single-strand nucleic acid conjugated with a fluorescent dye include a fluorescence quenching probe such as QPROBE® which is known as a guanine quenching probe used in the Example. A fluorescence quenching probe generally quenches fluorescence due to formation of double-strands and emits fluorescent light due to dissociating of the double-strands.

The detection signal representing the melting state of the nucleic acid mixture in the embodiments may be, for example, a signal generated due to non-melting of the sample and suppressed from generation due to melting of the sample as described above, or may be the opposite, a signal suppressed from generation due to non-melting of the sample and generated due to melting of the sample. The differential value of the detection signal may be, for example, a differential of the detection signal with respect to temperature expressed by (dF/dT), or may be expressed by (-dF/dT), where dF is the change in the detection signal and dT is the change in temperature. In a case in which detection signal generation is suppressed by melting of the sample, the peaks appear in valleys in the differential melting curve in which the detection signal differential values are expressed by (dF/dT), and the peaks appear in mountain shapes in the differential melting curve in which the detection signal differential values are expressed by (-dF/dT). In contrast, in cases in which the detection signal is generated by melting of the sample, the peaks appear in mountain shapes in the differential melting curve in which detection signal differential values are expressed by (dF/dT), and the peaks appear in valleys in the differential melting curve in which detection signal differential values are expressed by (-dF/dT). In both of these cases, the surface area of portions in specific temperature ranges bounded by the differential melting curve and a straight line representing base values can be derived. The differential value of the detection signal is not limited to a differential value of the detection signal differentiated with respect to temperature, and a differential value in which the detection signal is differentiated with respect to time may be employed.

Alternative examples which do not form part of the invention are shown in Fig. 10. For example, as shown in Fig. 10A, a ratio ($F_M/F_W$) which is the ratio of the detection signal level $F_M$ at the melting temperature $Tm_M$ to the detection signal level $F_W$ at the melting temperature $Tm_W$ may be employed as the ratio of characteristic amounts. Similarly, a ratio ($\Delta F_M/\Delta F_W$) which is the ratio of the detection signal level difference $\Delta F_M$ between the detection signal level at the lower limit of the temperature range $\Delta T_M$ and the detection signal level at the upper limit of the temperature range $\Delta T_M$ to the detection signal level difference $\Delta F_W$ between the detection signal level at the lower limit of the temperature range $\Delta T_W$ and the detection signal level at the upper limit of the temperature range $\Delta T_W$may be employed as the ratio of characteristic amounts. Further, a ratio ($(F - B)_M/(F- B)_W$), which is the ratio of $(F - B)_M$ that is the value of the detection signal level at the melting temperature $Tm_M$ from which the background level thereof has been subtracted to the value of $(F - B)_W$ that is the detection signal level at the melting temperature $Tm_W$ from which the background level thereof has been subtracted, may be employed as the ratio of characteristic amounts.

Further, as shown in Fig. 10B, a ratio ($f_M/f_W$) which is the ratio of the detection signal differential value $f_M$ at the melting temperature $Tm_M$ to the detection signal differential value $f_W$ at the melting temperature $Tm_W$ may be employed as the ratio of characteristic amounts. Similarly, a ratio ($\Sigma f_M/\Sigma f_W$) which is the ratio of $\Sigma f_M$, that is the sum of differential values of detection signal over the temperature range $\Delta T_M$ (corresponding to the surface area of the shaded portion slanting up to the right of Fig. 10B), to $\Sigma f_W$, that is the sum of differential values of detection signal over the temperature range $\Delta T_W$(corresponding to the surface area of the shaded portion slanting down to the right of Fig. 10B), may be employed as the ratio of characteristic amounts. Similarly, a ratio ($(f - B)_M/(f - B)_W$) which is the ratio of $(f - B)_M$, that is the value of the detection signal differential value at the melting temperature $Tm_M$ from which the background level thereof has been subtracted, to $(f - B)_W$, that is the value of the detection signal differential value at the melting temperature $Tm_W$ from which the background level thereof has been subtracted, may be employed as the ratio of characteristic amounts, $(f - B)_W$ can be derived by subtracting from $f_W$ a value corresponding to the melting temperature $Tm_W$ on a straight line passing through points corresponding to the lower limit and upper limit values of the temperature range $\Delta T_W$. Similarly, $(f - B)_M$ can be derived by subtracting from $f_M$ a value corresponding to the melting temperature $Tm_M$ on a straight line passing through points corresponding to the lower limit and upper limit values of the temperature range $\Delta T_M$.

In the above exemplary embodiment, measurement is made of an abundance ratio between nucleic acids that differ by only a single base. However, embodiments are not limited to the nucleic acids being homologous, and application may be made even to completely non-homologous nucleic acids as long as they have different melting temperatures from each other. For example, an abundance ratio can be determined between a gene A and a gene B for a nucleic acid mixture of non-homologous gene A and gene B having differing melting temperatures.

Whereas in the above exemplary embodiment, detection has been made by employing the same probe for each of the nucleic acids, different probes may be employed for each of the nucleic acids.

Further, in the above exemplary embodiment and Example, explanation is given of cases in which the temperature ranges respectively containing the melting temperatures of the nucleic acids ($\Delta T_W$ and $\Delta T_M$) are contiguous. However, as shown in Fig. 11, non-contiguous ranges can be set for the temperature ranges when the respective melting temperatures are separated from each other.

As an example not forming part of the invention, a fixed value may be employed as the base value for subtracting the background level, such as the differential value of the detection signal corresponding to either the upper limit value or the lower limit value of the temperature range. Nonetheless, as shown in Fig. 12, when there is a considerably difference between the detection signal differential values corresponding to the lower limit and upper limit values of the temperature range (when a shift in the base values of a specific amount or greater occurs), influence from the background can be removed with better precision by adopting the method of taking values on a straight line passing through points corresponding to the lower limit and the upper limit of the temperature range in the differential melting curve as the base values, as in the above exemplary embodiment and Example. Further, the base values may be determined on a curve instead of a straight line.

The above exemplary embodiment and Example describes cases in which a detection amount curve such as shown in Fig. 4 is employed as the detection amount data. However, embodiments are not limited thereto, and the detection amount data in which the relationship between a ratio of characteristic amounts and the abundance ratio of nucleic acids is expressed in a tabular format or in a computation formula may be employed.

In the above exemplary embodiment, the measurement result is displayed on the display section 14. However, embodiments are not limited thereto, and a printer may be provided and the measurement result may be printed out on a medium such as paper, the measurement result may be recorded on a portable recording medium, or may be output to an external device connected through the I/O port 28 and the network I/F 30.

The nucleic acid abundance ratio measurement device as described herein may be further provided with a determination section that determines progression of an illness of a patient based on a relationship between illness progression and nucleic acid abundance ratios that has been determined in advance, and on the computed nucleic acid abundance ratio.

The abundance ratio of mutated genes and normal genes may be measured and employed as a parameter that is considered when looking into a patient's condition. For example, a table may be generated in advance that defines relationship between proportions of mutated genes (for example genes related to malignant or cancer conditions) to normal genes in nucleic acids isolated from blood, and illness progression. The abundance ratio of mutant genes to normal genes of a patient may then be measured, and the illness progression can be assessed by comparison to the table generated in advance. Monitoring of a patient's condition, such as illness progression, can accordingly be performed by employing the determined nucleic acid abundance ratio.

The nucleic acid abundance ratio measurement device as described herein may be further provided with a determination section that determines at least one of constitution of a patient and/or drug administration amount appropriate for the constitution.

This determination is based on a predetermined relationship between the computed nucleic acid abundance ratio and constitution and/or drug administration amount appropriate for the constitution, and based on the computed nucleic acid abundance ratio of a patient. Constitution relates to the ease with which an investigation subject succumbs to a particular disease, and the effectiveness of a particular drug administration dose. For example, a table is generated in advance of a relationships between the proportion of a target gene of known copy number in a given gene (a gene with copy number variance), and factors as the ease of succumbing to a particular disease, the presence or absence of a particular disease, medical condition, drug effectiveness, drug administration amount and the like. The abundance ratio of the target gene in the gene with known copy number variance is determined and compared with the table that has been generated in advance. Thereby, the constitution, such as ease of succumbing to a particular disease and drug effectiveness, the presence or absence of a disease, medical condition, and appropriate drug administration amount can be determined. In this way, the determined abundance ratio can be employed to determine the constitution of an investigation subject and drug administration amount thereto. For example, subjects with a high copy number of CCL3L are known to have a constitution not readily susceptible to HIV, and subjects with a low copy number of FCGR3B are known to have a constitution readily susceptible to autoimmune diseases, such as systemic lupus erythematosus. Genes associated with conditions such as autism, schizophrenia, and sudden (certain) learning disabilities are also known to be expressed in association with high copy numbers.

Further, an embodiment may be configured as a nucleic acid abundance ratio measurement kit including a probe that can be hybridized with an area in a nucleic acid sequence that includes a target mutation, and a set of primers that can amplify the nucleic acid sequence including the target mutation. Using a measurement kit including such reagents may be advantageous in that target mutations of genes can be more easily detected and the abundance can be more easily measured.

Sequences of the probe and the primers that configure the nucleic acid abundance ratio measurement kit can be appropriately designed by a person skilled in the art, based on the sequence of the target gene and the sequence of the target mutated gene, if known. The length of the probe and annealing positions of the set of primers that are necessary to effectively implement testing can also be appropriately adjusted by a person skilled in the art.

It is preferable for the probe to be a labeled probe that is labeled from the standpoint of the efficiency of the testing. Specific examples of labeling material for the labeled probe include fluorescent dyes and fluorophores. A specific example of the labeled probe may preferably be a probe that has been labeled by a fluorescent dye and emits fluorescence independently, and in which the fluorescence emission decreases (quenches, for example) due to formation of a hybrid.

Probes using such a quenching phenomenon are generally referred to as fluorescence quenching probes.

Among them, a probe in which a base in the 3' region (3'-end) or the 5' region (5'-end) has been labeled by a fluorescent dye, and the labeled base is cytosine (C) is preferable. In this case, it is preferable to design the base sequence of the labeled probe such that a base in the testing target sequence which is to be paired with the terminal base (C) of the labeled probe, or a base that is separated by one to three bases from the base in the testing target sequence which is to be paired with the terminal base (C) of the labeled probe, is guanine (G). This kind of probe is generally referred to as a guanine quenching probe, which is known as a Q-Probe. When such a guanine quenching probe hybridizes with the testing target sequence, it exhibits a phenomenon such that fluorescence emission of the fluorescent dye is weakened (i.e., the intensity of the fluorescence decreases) due to the terminal base C which has been labeled by the fluorescent dye approaching the base G in the testing target sequence. By using such a probe, hybridization and disaggregation in the testing target sequence can be easily confirmed by variations in signals indicating the fluorescence emission. The labeling material can usually be conjugated to phosphate groups in a nucleotide.

Reagents included in the nucleic acid abundance ratio measurement kit of the present embodiment can be kept respectively in different containers, or be kept in the same container. The term "different containers" herein may be a container which is partitioned such that the reagents are maintained in non-contact states, and may not necessarily be separate containers that can be respectively handled independently.

The nucleic acid abundance ratio measurement kit of the present embodiment may further contain reagents or buffers necessary for amplification such as polymerase, reagents or buffers necessary for hybridization, diluents for diluting a testing subject, and the like. It is preferable for the nucleic acid abundance ratio measurement kit of the present embodiment to further contain an explanatory leaflet that explains the nucleic acid abundance ratio measurement method, an instruction leaflet regarding reagents that are included or can be additionally included in the kit, and the like.

Explanation in the above exemplary embodiments are given for cases in which melting curve data is acquired from an external instrument such as a melting curve analyzer, or acquired by reading data stored on a storage medium. However, the melting curve analyzing device may be integrated together with the nucleic acid abundance ratio measurement device described herein. Specifically, nucleic acid abundance ratio measurement device may include a temperature controller that controls temperature change of the nucleic acid mixture, and a measurement section for measuring a detection signal such as degree of light absorption, fluorescence intensity, and relative fluorescence, in addition to the configuration described hereinbefore. Data of the melting curve may be obtained by the measurement section by measuring the detection signal while the temperature of the nucleic acid mixture of the test sample is being changed by the temperature controller. The nucleic acid abundance ratio of the sample can then subsequently be determined in the same manner to that of the above exemplary embodiments.

The above nucleic acid abundance ratio measurement routine may be provided via a storage medium storing a program thereof.

<Appendix>

## Claims

**1.** A nucleic acid abundance ratio measurement device (10) comprising:

a detection section suitable for detecting a detection signal over different temperature ranges of a melting curve for a nucleic acid mixture having two nucleic acids having different melting temperatures; and

an abundance ratio computation section configured to compute the nucleic acid abundance ratio of the two nucleic acids in the nucleic acid mixture based on the ratio of characteristic amounts for the nucleic acids obtained from the detection signal detected by the detection section and based on detection amount data which express the relationship between the ratios of characteristic amounts of the nucleic acids, obtained from the detection signal of the melting curves over two temperature ranges respectively containing the different melting temperatures, and nucleic acid abundance ratios, wherein the detection amount data is based on respective differential melting curves each expressing a relationship between temperature and a detection signal obtained from each of a plurality of nucleic acid mixtures of different abundance ratios of the two nucleic acids having different melting temperatures,

wherein the characteristic amount ratio is set as the ratio, in a differential melting curve expressing a relationship between temperature and differential values of the detection signal, between a first surface area of a region bounded by a straight line passing through a point of the differential melting curve corresponding to the lower limit value of a first temperature range and a point of the differential melting curve

corresponding to the upper limit value of the first temperature range and bounded by the melting curve, and a second surface area of a region bounded by a straight line passing through a point of the differential melting curve corresponding to the lower limit value of a second temperature range and a point of the differential melting curve corresponding to the upper limit value of the second temperature range and bounded by the melting curve.

**2.** The device as claimed in claim 1, further comprising:

a storage section (26) for storing the detection amount data; or

an input section (28) for inputting the detection amount data to the abundance ratio computation section.

**3.** The device as claimed in claim 1 or 2, wherein the abundance ratio computation section is configured to select the lower limit values or the upper limit values of the first and second temperature ranges from:

temperatures at which the differential value is a minimum value between two peaks of the differential melting curve,
temperatures corresponding to temperature values at the tails of the peak of the differential melting curve, or temperatures within ±15°C of the temperature corresponding to the peak of the differential melting curve.

**4.** The device as claimed in claim 1 or 2, wherein the nuclear abundance ratio computation section is configured to select the lower limit values or the upper limit values of the first and second temperature ranges as temperatures within ±10°C, preferably ± 7°C, of the temperature corresponding to the peak of the differential melting curve.

**5.** The device as claimed in any of claims 1-4, wherein the nuclear abundance ratio computation section is configured to select the upper limit value of one of the first or second temperature range as different from the lower limit value of the other of the first or second temperature range that is a higher range than that of the one of the first or second temperature range.

**6.** The device as claimed in any one of claims 1-5, wherein the nuclear abundance ratio computation section is configured to set the width of the first temperature range from its lower limit value to its upper limit value and the width of the second temperature range from its lower limit value to its upper limit value as identical.

**7.** The device as claimed in any one of claims 1-5, wherein the nuclear abundance ratio computation section is configured to set the width of the first temperature range from its lower limit value to its upper limit value and the width of the second temperature range from its lower limit value to its upper limit value as different.

**8.** The device as claimed in any one of claims 1-7, further comprising:

a temperature controller suitable for controlling the change in temperature of the nucleic acid mixture; and
a measurement section suitable for measuring the degree of light absorption, fluorescence intensity or relative fluorescence intensity as the detection signal.

**9.** A nucleic acid abundance ratio measurement method comprising:

detecting a detection signal over different temperature ranges for a melting curve of a target nucleic acid mixture having two nucleic acids having different melting temperatures; and
computing the nucleic acid abundance ratio of the two nucleic acids in the target nucleic acid mixture based on the ratio of characteristic amounts for the nucleic acids obtained from the detection signal that has been detected and based on detection amount data which express the relationship between the ratios of characteristic amounts of the nucleic acids, which detection amount data have been obtained from the detection signal of the melting curves over two temperature ranges respectively containing the different melting temperatures, and nucleic acid abundance ratios, wherein the detection amount data is based on respective differential melting curves each expressing a relationship between temperature and a detection signal which melting curves have been obtained from each of a plurality of nucleic acid mixtures of different abundance ratios of the two nucleic acids having different melting temperatures,
wherein the characteristic amount ratio is set as the ratio, in a differential melting curve expressing a relationship between temperature and differential values of the detection signal, between a first surface area of a region bounded by a straight line passing through a point of the differential melting curve corresponding to the lower limit value of a first temperature range and a point of the differential melting curve corresponding to the upper limit value of the first temperature range and bounded by the melting curve, and a second surface area of a region bounded by a straight line passing through a point of the differential melting curve corresponding to the lower limit value of a second temperature range and a point of the differential melting curve corresponding to the upper limit value of the second temperature range and bounded by the melting curve.

**10.** The nucleic acid abundance ratio measurement method as claimed in claim 9, comprising:

generating the detection amount data;
computing said ratio of characteristic amounts of the nucleic acids in the target nucleic acid mixture based on the detection signal of the melting curve of the target nucleic acid mixture over temperature ranges corresponding to each of the two respective temperature ranges; and
computing said nucleic acid abundance ratio of the nucleic acids in the target nucleic acid mixture based on the computed ratio of characteristic amounts and the generated detection amount data.

**11.** A non-transitory storage medium storing a program that causes a computer (16) to execute abundance ratio measurement processing, the abundance ratio measurement processing comprising:

receiving a detection signal of a melting curve of a target nucleic acid mixture having two nucleic acids having different melting temperatures, the detection signal being detected over different temperature ranges; and
computing the nucleic acid abundance ratio of the two nucleic acids in the target nucleic acid mixture based on the ratio of characteristic amounts for the nucleic acids obtained from the received detection signal and based on detection amount data which express the relationship between the ratios of characteristic amounts of the nucleic acids, obtained from the detection signal of the melting curves over two temperature ranges respectively containing the different melting temperatures, and nucleic acid abundance ratios, wherein the detection amount data is based on respective differential melting curves each expressing a relationship between temperature and a detection signal obtained from each of a plurality of nucleic acid mixtures of different abundance ratios of the two nucleic acids having different melting temperatures,
wherein the characteristic amount ratio is set as the ratio, in a differential melting curve expressing a relationship between temperature and differential values of the detection signal, between a first surface area of a region bounded by a straight line passing through a point of the differential melting curve corresponding to the lower limit value of a first temperature range and a point of the differential melting curve corresponding to the upper limit value of the first temperature range and bounded by the melting curve, and a second surface area of a region bounded by a straight line passing through a point of the differential melting curve corresponding to the lower limit value of a second temperature range and a point of the differential melting curve corresponding to the upper limit value of the second temperature range and bounded by the melting curve.

**12.** The non-transitory storage medium as claimed in claim 11, the abundance ratio measurement processing further comprising:

acquiring the detection amount data from a storage section that stores the detection amount data, or acquiring the detection amount data input by an input section;
computing said ratio of characteristic amounts of the nucleic acids in the target nucleic acid mixture based on the detection signal of the melting curve of the target nucleic acid mixture, the detection signal being detected over temperature ranges respectively corresponding to the two temperature ranges; and
computing said nucleic acid abundance ratio of the nucleic acids in the target nucleic acid mixture based on the computed ratio of characteristic amounts and the acquired detection amount data.

**13.** A determination method of determining the condition of a patient, comprising:

obtaining a nucleic acid abundance ratio with the device of any one of claims 1-8, by the method of claim 9 or 10, or by executing the program as defined in claim 11 or 12; and
determining said condition based on said nucleic acid abundance ratio by comparison to a relationship, wherein the relationship is the relationship between the nucleic acid abundance ratios and the condition of the patient and has been determined in advance of the determination method.

**14.** A determination method of determining the constitution of a patient and/or an appropriate drug administration amount for the constitution, comprising:

obtaining a nucleic acid abundance ratio with the device of any one of claims 1-8, by the method of claim 9 or 10, or by executing the program as defined in claim 11 or 12; and
determining said constitution of the patient and/or the appropriate drug administration amount for the constitution, based on said nucleic acid abundance ratio by comparison to a relationship, wherein the relationship is the relationship between the nucleic acid abundance ratios and the constitutions and/or appropriate drug administration amount for the constitutions and has been determined in advance of the determination method.

**15.** Use of a nucleic acid abundance ratio measurement kit in the measurement of a nucleic acid abundance ratio using the device of any one of claims 1-8, the method of claim 9 or 10, or the program as defined in claim 11 or 12, the kit comprising:

a probe that can be hybridized with a region of a nucleic acid sequence that includes a target mutation that may exist in the nucleic acid mixture, the probe preferably being a labeled probe; and
a set of primers that can amplify the nucleic acid sequence that includes the target mutation.

## Patentansprüche

1. Nukleinsäurehäufigkeitsverhältnismessungsvorrichtung (10), umfassend:

   einen Detektionsabschnitt, der zum Detektieren eines Detektionssignals über unterschiedliche Temperaturspannen einer Schmelzkurve für ein Nukleinsäuregemisch geeignet ist, das zwei Nukleinsäuren aufweist, die unterschiedliche Schmelztemperaturen aufweisen; und
   einen Häufigkeitsverhältnisberechnungsabschnitt, konfiguriert, um das Nukleinsäurehäufigkeitsverhältnissen der zwei Nukleinsäuren in dem Nukleinsäuregemisch basierend auf dem Verhältnis von charakteristischen Mengen für die Nukleinsäuren, die aus dem Detektionssignal erhalten werden, das von dem Detektionsabschnitt detektiert wird, und basierend auf Detektionsmengendaten, die die Beziehung zwischen den Verhältnissen von charakteristischen Mengen der Nukleinsäuren, die aus dem Detektionssignal der Schmelzkurven über zwei Temperaturspannen erhalten werden, die jeweils die unterschiedlichen Schmelztemperaturen beinhalten, und Nukleinsäurehäufigkeitsverhältnissen ausdrücken, zu errechnen, wobei die Detektionsmengendaten auf jeweiligen Differentialschmelzkurven basieren, wobei jede eine Beziehung zwischen Temperatur und einem Detektionssignal ausdrückt, das von jedem einer Vielzahl von Nukleinsäuregemischen unterschiedlicher Häufigkeitsverhältnisse der zwei Nukleinsäuren erhalten wird, die unterschiedliche Schmelztemperaturen aufweisen,
   wobei das charakteristische Mengenverhältnis als das Verhältnis in einer Differentialschmelzkurve festgelegt ist, das eine Beziehung zwischen Temperatur- und Differentialwerten des Detektionssignals zwischen einem ersten Flächenbereich eines Gebiets, das von einer geraden Linie begrenzt ist, die durch einen Punkt der Differentialschmelzkurve, der dem unteren Grenzwert einer ersten Temperaturspanne entspricht, und einen Punkt der Differentialschmelzkurve, der dem oberen Grenzwert der ersten Temperaturspanne entspricht und durch die Schmelzkurve begrenzt ist, verläuft, und einem zweiten Flächenbereich eines Gebiets ausdrückt, das durch eine gerade Linie begrenzt ist, die durch einen Punkt der Differentialschmelzkurve, der dem unteren Grenzwert einer zweiten Temperaturspanne entspricht, und einen Punkt der Differentialschmelzkurve, der dem oberen Grenzwert der zweiten Temperaturspanne entspricht und durch die Schmelzkurve begrenzt ist, verläuft.

2. Vorrichtung nach Anspruch 1, weiter umfassend:

   einen Speicherabschnitt (26) zum Speichern der Detektionsmengendaten; oder
   einen Eingabeabschnitt (28) zum Eingeben der Detektionsmengendaten zu dem Häufigkeitsverhältnisberechnungsabschnitt.

3. Vorrichtung nach Anspruch 1 oder 2, wobei der Häufigkeitsverhältnisberechnungsabschnitt konfiguriert ist, um die unteren Grenzwerte oder die oberen Grenzwerte der ersten und zweiten Temperaturspanne auszuwählen aus:

   Temperaturen, bei denen der Differentialwert ein minimaler Wert zwischen zwei Spitzen der Differentialschmelzkurve ist,
   Temperaturen entsprechend Temperaturwerten bei den Ausläufern der Spitzen der Differentialschmelzkurve, oder
   Temperaturen innerhalb von ±15 °C der Temperatur, entsprechend der Spitze der Differentialschmelzkurve.

4. Vorrichtung nach Anspruch 1 oder 2, wobei der nukleare Häufigkeitsverhältnisberechnungsabschnitt konfiguriert ist, um die unteren Grenzwerte oder die oberen Grenzwerte der ersten und zweiten Temperaturspanne als Temperaturen innerhalb von ±10°C, vorzugsweise ±7 °C, der Temperatur entsprechend der Spitze der Differentialschmelzkurve auszuwählen.

5. Vorrichtung nach einem der Ansprüche 1-4, wobei der nukleare Häufigkeitsverhältnisberechnungsabschnitt konfiguriert ist, um den oberen Grenzwert einer der ersten oder zweiten Temperaturspanne als unterschiedlich von dem unteren Grenzwert der anderen der ersten oder zweiten Temperaturspanne, die eine höhere Spanne als die der einen der ersten oder zweiten Temperaturspanne ist, auszuwählen.

6. Vorrichtung nach einem der Ansprüche 1-5, wobei der nukleare Häufigkeitsverhältnisberechnungsabschnitt konfiguriert ist, um die Breite der ersten Temperaturspanne von ihrem unteren Grenzwert zu ihrem oberen Grenzwert und die Breite der zweiten Temperaturspanne von ihrem unteren Grenzwert zu ihrem oberen Grenzwert als identisch festzusetzen.

7. Vorrichtung nach einem der Ansprüche 1-5, wobei der nukleare Häufigkeitsverhältnisberechnungsabschnitt konfiguriert ist, um die Breite der ersten Temperaturspanne von ihrem unteren Grenzwert zu ihrem oberen Grenzwert und die Breite der zweiten Temperaturspanne von ihrem unteren Grenzwert zu ihrem oberen Grenzwert als unterschiedlich festzusetzen.

**8.** Vorrichtung nach einem der Ansprüche 1-7, weiter umfassend:

eine Temperatursteuerung, die zum Steuern der Temperaturänderung des Nukleinsäuregemisches geeignet ist; und
einen Messungsabschnitt, der zum Messen des Lichtabsorptionsgrads, der Fluoreszenzintensität oder relativen Fluoreszenzintensität als das Detektionssignal geeignet ist.

**9.** Nukleinsäurehäufigkeitsverhältnismessungsverfahren, umfassend:

Detektieren eines Detektionssignals über unterschiedliche Temperaturspannen für eine Schmelzkurve eines Ziel-Nukleinsäuregemisches, das zwei Nukleinsäuren aufweist, die unterschiedliche Schmelztemperaturen aufweisen; und
Berechnen des Nukleinsäurehäufigkeitsverhältnisses der zwei Nukleinsäuren in dem Ziel-Nukleinsäuregemisch, basierend auf dem Verhältnis von charakteristischen Mengen für die Nukleinsäuren, die aus dem Detektionssignal erhalten werden, das detektiert wurde, und basierend auf Detektionsmengendaten, die die Beziehung zwischen den Verhältnissen von charakteristischen Mengen der Nukleinsäuren ausdrücken, wobei die Detektionsmengendaten aus dem Detektionssignal der Schmelzkurven über zwei Temperaturspannen erhalten wurden, die jeweils die unterschiedlichen Schmelztemperaturen beinhalten, und Nukleinsäurehäufigkeitsverhältnissen, wobei die Detektionsmengendaten auf jeweiligen Differentialschmelzkurven basieren, wobei jede eine Beziehung zwischen Temperatur und einem Detektionssignal ausdrückt, wobei die Schmelzkurven aus jedem einer Vielzahl von Nukleinsäuregemischen unterschiedlicher Häufigkeitsverhältnisse der zwei Nukleinsäuren erhalten wurden, die unterschiedliche Schmelztemperaturen aufweisen,
wobei das charakteristische Mengenverhältnis als das Verhältnis in einer Differentialschmelzkurve festgelegt ist, das eine Beziehung zwischen Temperatur- und Differentialwerten des Detektionssignals zwischen einem ersten Flächenbereich eines Gebiets, das von einer geraden Linie begrenzt ist, die durch einen Punkt der Differentialschmelzkurve, der dem unteren Grenzwert einer ersten Temperaturspanne entspricht, und einen Punkt der Differentialschmelzkurve, der dem oberen Grenzwert der ersten Temperaturspanne entspricht und durch die Schmelzkurve begrenzt ist, verläuft, und einem zweiten Flächenbereich eines Gebiets ausdrückt, das durch eine gerade Linie begrenzt ist, die durch einen Punkt der Differentialschmelzkurve, der dem unteren Grenzwert einer zweiten Temperaturspanne entspricht, und einen Punkt der Differentialschmelzkurve, der dem oberen Grenzwert der zweiten Temperaturspanne entspricht und durch die Schmelzkurve begrenzt ist, verläuft.

**10.** Nukleinsäurehäufigkeitsverhältnismessungsverfahren nach Anspruch 9, umfassend:

Erzeugen der Detektionsmengendaten;
Berechnen des Verhältnisses von charakteristischen Mengen der Nukleinsäuren in dem Ziel-Nukleinsäuregemisch, basierend auf dem Detektionssignal der Schmelzkurve des Ziel-Nukleinsäuregemisches über Temperaturspannen, die jeder der zwei jeweiligen Temperaturspannen entsprechen; und
Berechnen des Nukleinsäurehäufigkeitsverhältnisses der Nukleinsäuren in dem Ziel-Nukleinsäuregemisch, basierend auf dem berechneten Verhältnis von charakteristischen Mengen und den erzeugten Detektionsmengendaten.

**11.** Nichtflüchtiges Speichermedium, das ein Programm speichert, das einen Computer (16) veranlasst, Häufigkeitsverhältnismessungsverarbeitung auszuführen, wobei die Häufigkeitsverhältnismessungsverarbeitung umfasst:

Empfangen eines Detektionssignals einer Schmelzkurve eines Ziel-Nukleinsäuregemisches, das zwei Nukleinsäuren aufweist, die unterschiedliche Schmelztemperaturen aufweisen, wobei das Detektionssignal über unterschiedliche Temperaturspannen detektiert wird; und
Berechnen des Nukleinsäurehäufigkeitsverhältnisses der zwei Nukleinsäuren in dem Ziel-Nukleinsäuregemisch, basierend auf dem Verhältnis von charakteristischen Mengen für die Nukleinsäuren, das aus dem empfangenen Detektionssignal erhalten wird, und basierend auf Detektionsmengendaten, die die Beziehung zwischen den Verhältnissen von charakteristischen Mengen der Nukleinsäuren ausdrücken, die aus dem Detektionssignal der Schmelzkurven über zwei Temperaturspannen erhalten werden, die jeweils die unterschiedlichen Schmelztemperaturen beinhalten, und Nukleinsäurehäufigkeitsverhältnissen, wobei die Detektionsmengendaten auf jeweiligen Differentialschmelzkurven basieren, wobei jedes eine Beziehung zwischen Temperatur und einem Detektionssignal ausdrückt, die aus jedem einer Vielzahl von Nukleinsäuregemischen unterschiedlicher Häufigkeitsverhältnisse der zwei Nukleinsäuren erhalten werden, die unterschiedliche Schmelztemperaturen aufweisen,
wobei das charakteristische Mengenverhältnis als das Verhältnis in einer Differentialschmelzkurve festgelegt ist, das eine Beziehung zwischen Temperatur- und Differentialwerten des Detektionssignals zwischen einem ersten Flächenbereich eines Gebiets, das von einer geraden Linie begrenzt ist, die durch einen Punkt der Differentialschmelzkurve, der dem unteren Grenzwert einer ersten Temperaturspanne entspricht, und einen Punkt der Differentialschmelzkurve, der dem oberen Grenzwert der ersten Temperaturspanne entspricht und durch die Schmelzkurve begrenzt ist, verläuft, und einem zweiten Flächenbereich eines Gebiets ausdrückt, das durch eine gerade Linie begrenzt ist, die durch einen Punkt der Differentialschmelzkurve, der dem unteren Grenzwert einer zweiten Temperaturspanne entspricht, und einen Punkt der Differentialschmelzkurve, der dem oberen Grenzwert der zweiten Temperaturspanne entspricht und durch die Schmelzkurve begrenzt ist, verläuft.

**12.** Nichtflüchtiges Speichermedium nach Anspruch 11, wobei die Häufigkeitsverhältnismessungsverarbeitung weiter umfasst:

Akquirieren der Detektionsmengendaten aus einem Speicherabschnitt, der die Detektionsmengendaten speichert, oder Akquirieren der Detektionsmengendaten, die durch einen Eingabeabschnitt eingegeben werden;
Berechnen des Verhältnisses von charakteristischen Mengen der Nukleinsäuren in dem Ziel-Nukleinsäuregemisch, basierend auf dem Detektionssignal der Schmelzkurve des Ziel-Nukleinsäuregemisches, wobei das Detektionssignal über Temperaturspannen detektiert wird, die jeweils den zwei Temperaturspannen entsprechen; und
Berechnen des Nukleinsäurehäufigkeitsverhältnisses der Nukleinsäuren in dem Ziel-Nukleinsäuregemisch, basierend auf dem berechneten Verhältnis von charakteristischen Mengen und den akquirierten Detektionsmengendaten.

**13.** Ermittlungsverfahren zum Ermitteln des Zustands eines Patienten, umfassend:

Erhalten eines Nukleinsäurehäufigkeitsverhältnisses mit der Vorrichtung nach einem der Ansprüche 1-8, durch das Verfahren nach Anspruch 9 oder 10, oder durch Ausführen des Programms nach Anspruch 11 oder 12; und
Ermitteln des Zustands, basierend auf dem Nukleinsäurehäufigkeitsverhältnis durch Vergleich mit einer Beziehung, wobei die Beziehung die Beziehung zwischen den Nukleinsäurehäufigkeitsverhältnissen und dem Zustand des Patienten ist und vor dem Ermittlungsverfahren ermittelt wurde.

**14.** Ermittlungsverfahren zum Ermitteln der Konstitution eines Patienten und/oder einer angemessenen Arzneiverabreichungsmenge für die Konstitution, umfassend:

Erhalten eines Nukleinsäurehäufigkeitsverhältnisses mit der Vorrichtung nach einem der Ansprüche 1-8, durch das Verfahren nach Anspruch 9 oder 10 oder durch Ausführen des Programms nach Anspruch 11 oder 12; und
Ermitteln der Konstitution des Patienten und/oder der angemessenen Arzneiverabreichungsmenge für die Konstitution, basierend auf dem Nukleinsäurehäufigkeitsverhältnis durch Vergleich mit einer Beziehung, wobei die Beziehung die Beziehung zwischen den Nukleinsäurehäufigkeitsverhältnissen und den Konstitutionen und/oder angemessener Arzneiverabreichungsmenge für die Konstitutionen ist und vor dem Ermittlungsverfahren ermittelt wurde.

**15.** Verwendung eines Nukleinsäurehäufigkeitsverhältnissen-Messungskits bei der Messung eines Nukleinsäurehäufigkeitsverhältnisses unter Verwendung der Vorrichtung nach einem der Ansprüche 1-8, des Verfahrens nach Anspruch 9 oder 10 oder dem Programm nach Anspruch 11 oder 12, wobei das Kit umfasst:

eine Sonde, die mit einem Bereich einer Nukleinsäuresequenz hybridisiert werden kann, der eine Zielmutation einschließt, die in dem Nukleinsäuregemisch bestehen kann, wobei die Sonde vorzugsweise eine markierte Sonde ist; und
einen Satz von Primern, die die Nukleinsäure Sequenz amplifizieren können, die die Zielmutation einschließt.

## Revendications

1. **1.** Dispositif de mesure de rapport d'abondance d'acides nucléiques (10) comprenant :

   une section de détection convenant pour détecter un signal de détection sur des plages de températures différentes d'une courbe de fusion pour un mélange d'acides nucléiques ayant deux acides nucléiques ayant des températures de fusion différentes ; et
   une section de calcul de rapport d'abondance configurée pour calculer le rapport d'abondance d'acides nucléiques des deux acides nucléiques dans le mélange d'acides nucléiques d'après le rapport de quantités caractéristiques pour les acides nucléiques obtenu à partir du signal de détection détecté par la section de détection et d'après des données de quantité de détection qui expriment la relation entre les rapports de quantités caractéristiques des acides nucléiques, obtenues à partir du signal de détection des courbes de fusion sur deux plages de températures contenant respectivement les températures de fusion différentes, et des rapports d'abondance d'acides nucléiques, dans lequel les données de quantité de détection sont basées sur des courbes de fusion différentielles respectives exprimant chacune une relation entre la température et un signal de détection obtenues à partir de chacun d'une pluralité de mélanges d'acides nucléiques de rapports d'abondance différents des deux acides nucléiques ayant des températures de fusion différentes,
   dans lequel le rapport de quantités caractéristiques est fixé comme le rapport, dans une courbe de fusion différentielle exprimant une relation entre la température et des valeurs différentielles du signal de détection, entre une première aire d'une région délimitée par une droite passant par un point de la courbe de fusion différentielle correspondant à la valeur limite inférieure d'une première plage de températures et un point de la courbe de fusion différentielle correspondant à la valeur limite supérieure de la première plage de températures et délimitée par la courbe de fusion, et une seconde aire d'une région délimitée par une droite passant par un point de la courbe de fusion différentielle correspondant à la valeur limite inférieure d'une seconde plage de températures et un point de la courbe de fusion différentielle correspondant à la valeur limite supérieure de la seconde plage de températures et délimitée par la courbe de fusion.

2. **2.** Dispositif selon la revendication 1, comprenant en outre :

   une section de stockage (26) destinée à stocker les données de quantité de détection ; ou
   une section d'entrée (28) destinée à fournir les données de quantité de détection en entrée à la section de calcul de rapport d'abondance.

3. **3.** Dispositif selon la revendication 1 ou 2, dans lequel la section de calcul de rapport d'abondance est configurée pour sélectionner les valeurs limites inférieures ou les valeurs limites supérieures des première et seconde plages de températures à partir :

   de températures auxquelles la valeur différentielle est une valeur minimale entre deux pics de la courbe de fusion différentielle,
   de températures correspondant à des valeurs de température au niveau des bases du pic de la courbe de fusion différentielle, ou
   de températures à ± 15 °C de la température correspondant au pic de la courbe de fusion différentielle.

4. **4.** Dispositif selon la revendication 1 ou 2, dans lequel la section de calcul de rapport d'abondance nucléaire est configurée pour sélectionner les valeurs limites inférieures ou les valeurs limites supérieures des première et seconde plages de températures comme des températures à ± 10 °C, de préférence ± 7 °C, de la température correspondant au pic de la courbe de fusion différentielle.

5. **5.** Dispositif selon l'une quelconque des revendications 1 à 4, dans lequel la section de calcul de rapport d'abondance nucléaire est configurée pour sélectionner la valeur limite supérieure de l'une de la première ou seconde plage de températures comme différente de la valeur limite inférieure de l'autre de la première ou seconde plage de températures qui est une plage plus élevée que celle de l'une de la première ou seconde plage de températures.

6. **6.** Dispositif selon l'une quelconque des revendications 1 à 5, dans lequel la section de calcul de rapport d'abondance nucléaire est configurée pour fixer la largeur de la première plage de températures de sa valeur limite inférieure à sa valeur limite supérieure et la largeur de la seconde plage de températures de sa valeur limite inférieure à sa valeur limite supérieure comme identiques.

**7.** Dispositif selon l'une quelconque des revendications 1 à 5, dans lequel la section de calcul de rapport d'abondance nucléaire est configurée pour fixer la largeur de la première plage de températures de sa valeur limite inférieure à sa valeur limite supérieure et la largeur de la seconde plage de températures de sa valeur limite inférieure à sa valeur limite supérieure comme différentes.

**8.** Dispositif selon l'une quelconque des revendications 1 à 7, comprenant en outre :

un régulateur de température convenant pour réguler le changement de température du mélange d'acides nucléiques ; et
une section de mesure convenant pour mesurer le degré d'absorption de lumière, l'intensité de fluorescence ou l'intensité de fluorescence relative comme signal de détection.

**9.** Procédé de mesure de rapport d'abondance d'acides nucléiques comprenant :

la détection d'un signal de détection sur des plages de températures différentes pour une courbe de fusion d'un mélange d'acides nucléiques cible ayant deux acides nucléiques ayant des températures de fusion différentes ; et
le calcul du rapport d'abondance d'acides nucléiques des deux acides nucléiques dans le mélange d'acides nucléiques cible d'après le rapport de quantités caractéristiques pour les acides nucléiques obtenu à partir du signal de détection qui a été détecté et d'après des données de quantité de détection qui expriment la relation entre les rapports de quantités caractéristiques des acides nucléiques, lesquelles données de quantité de détection ont été obtenues à partir du signal de détection des courbes de fusion sur deux plages de températures contenant respectivement les températures de fusion différentes, et des rapports d'abondance d'acides nucléiques, dans lequel les données de quantité de détection sont basées sur des courbes de fusion différentielles respectives exprimant chacune une relation entre la température et un signal de détection dont des courbes de fusion ont été obtenues à partir de chacun d'une pluralité de mélanges d'acides nucléiques de rapports d'abondance différents des deux acides nucléiques ayant des températures de fusion différentes,
dans lequel le rapport de quantités caractéristiques est fixé comme le rapport, dans une courbe de fusion différentielle exprimant une relation entre la température et des valeurs différentielles du signal de détection, entre une première aire d'une région délimitée par une droite passant par un point de la courbe de fusion différentielle correspondant à la valeur limite inférieure d'une première plage de températures et un point de la courbe de fusion différentielle correspondant à la valeur limite supérieure de la première plage de températures et délimitée par la courbe de fusion, et une seconde aire d'une région délimitée par une droite passant par un point de la courbe de fusion différentielle correspondant à la valeur limite inférieure d'une seconde plage de températures et un point de la courbe de fusion différentielle correspondant à la valeur limite supérieure de la seconde plage de températures et délimitée par la courbe de fusion.

**10.** Procédé de mesure de rapport d'abondance d'acides nucléiques selon la revendication 9, comprenant :

la génération des données de quantité de détection ;
le calcul dudit rapport de quantités caractéristiques des acides nucléiques dans le mélange d'acides nucléiques cible d'après le signal de détection de la courbe de fusion du mélange d'acides nucléiques cible sur des plages de températures correspondant à chacune des deux plages de températures respectives ; et
le calcul dudit rapport d'abondance d'acides nucléiques des acides nucléiques dans le mélange d'acides nucléiques cible d'après le rapport calculé de quantités caractéristiques et les données de quantité de détection générées.

**11.** Support de stockage non transitoire stockant un programme qui amène un ordinateur (16) à exécuter un traitement de mesure de rapport d'abondance, le traitement de mesure de rapport d'abondance comprenant :

la réception d'un signal de détection d'une courbe de fusion d'un mélange d'acides nucléiques cible ayant deux acides nucléiques ayant des températures de fusion différentes, le signal de détection étant détecté sur des plages de températures différentes ; et
le calcul du rapport d'abondance d'acides nucléiques des deux acides nucléiques dans le mélange d'acides nucléiques cible d'après le rapport de quantités caractéristiques pour les acides nucléiques obtenu à partir du signal de détection reçu et d'après des données de quantité de détection qui expriment la relation entre les rapports de quantités caractéristiques des acides nucléiques, obtenues à partir du signal de détection des courbes de fusion sur deux plages de températures contenant respectivement les températures de fusion différentes, et des rapports d'abondance d'acides nucléiques, dans lequel les données de quantité de détection sont basées sur des courbes de fusion différentielles respectives exprimant chacune une relation entre la température et un signal de détection obtenues à partir de chacun d'une pluralité de mélanges d'acides nucléiques de rapports d'abondance différents des deux acides nucléiques ayant des températures de fusion différentes,
dans lequel le rapport de quantités caractéristiques est fixé comme le rapport, dans une courbe de fusion différentielle exprimant une relation entre la température et des valeurs différentielles du signal de détection, entre une première aire d'une région délimitée par une droite passant par un point de la courbe de fusion différentielle correspondant à la valeur limite inférieure d'une première plage de températures et un point de la courbe de fusion différentielle correspondant à la valeur limite supérieure de la première plage de températures et délimitée par la courbe de fusion, et une seconde aire d'une région délimitée par une droite passant par un point de la courbe de fusion différentielle correspondant à la valeur limite inférieure d'une seconde plage de températures et un point de la courbe de fusion différentielle correspondant à la valeur limite supérieure de la seconde plage de températures et délimitée par la courbe de fusion.

**12.** Support de stockage non transitoire selon la revendication 11, le traitement de mesure de rapport d'abondance comprenant en outre :

l'acquisition des données de quantité de détection auprès d'une section de stockage qui stocke les données de quantité de détection, ou l'acquisition des données de quantité de détection fournies en entrée par une section d'entrée ;
le calcul dudit rapport de quantités caractéristiques des acides nucléiques dans le mélange d'acides nucléiques cible d'après le signal de détection de la courbe de fusion du mélange d'acides nucléiques cible, le signal de détection étant détecté sur des plages de températures correspondant respectivement aux deux plages de températures ; et
le calcul dudit rapport d'abondance d'acides nucléiques des acides nucléiques dans le mélange d'acides nucléiques cible d'après le rapport calculé de quantités caractéristiques et les données de quantité de détection acquises.

**13.** Procédé de détermination consistant à déterminer l'état d'un patient, comprenant :

l'obtention d'un rapport d'abondance d'acides nucléiques avec le dispositif de l'une quelconque des revendications 1 à 8, par le procédé de la revendication 9 ou 10, ou par exécution du programme tel que défini dans la revendication 11 ou 12 ; et
la détermination dudit état d'après ledit rapport d'abondance d'acides nucléiques par comparaison à une relation, dans lequel la relation est la relation entre les rapports d'abondance d'acides nucléiques et l'état du patient et a été déterminée préalablement au procédé de détermination.

**14.** Procédé de détermination consistant à déterminer la constitution d'un patient et/ou une quantité appropriée d'administration de médicament pour la constitution, comprenant :

l'obtention d'un rapport d'abondance d'acides nucléiques avec le dispositif de l'une quelconque des revendications 1 à 8, par le procédé de la revendication 9 ou 10, ou par exécution du programme tel que défini dans la revendication 11 ou 12 ; et
la détermination de ladite constitution du patient et/ou de la quantité appropriée d'administration de médicament pour la constitution, d'après ledit rapport d'abondance d'acides nucléiques par comparaison à une relation, dans lequel la relation est la relation entre les rapports d'abondance d'acides nucléiques et les constitutions et/ou la quantité appropriée d'administration de médicament pour les constitutions et a été déterminée préalablement au procédé de détermination.

**15.** Utilisation d'un nécessaire de mesure de rapport d'abondance d'acides nucléiques dans la mesure d'un rapport d'abondance d'acides nucléiques utilisant le dispositif de l'une quelconque des revendications 1 à 8, le procédé de la revendication 9 ou 10, ou le programme tel que défini dans la revendication 11 ou 12, le nécessaire comprenant :

une sonde qui peut être hybridée avec une région d'une séquence d'acides nucléiques qui inclut une mutation cible qui peut exister dans le mélange d'acides nucléiques, la sonde étant de préférence une sonde marquée ; et
un jeu d'amorces qui peut amplifier la séquence d'acides nucléiques qui inclut la mutation cible.

FIG.1

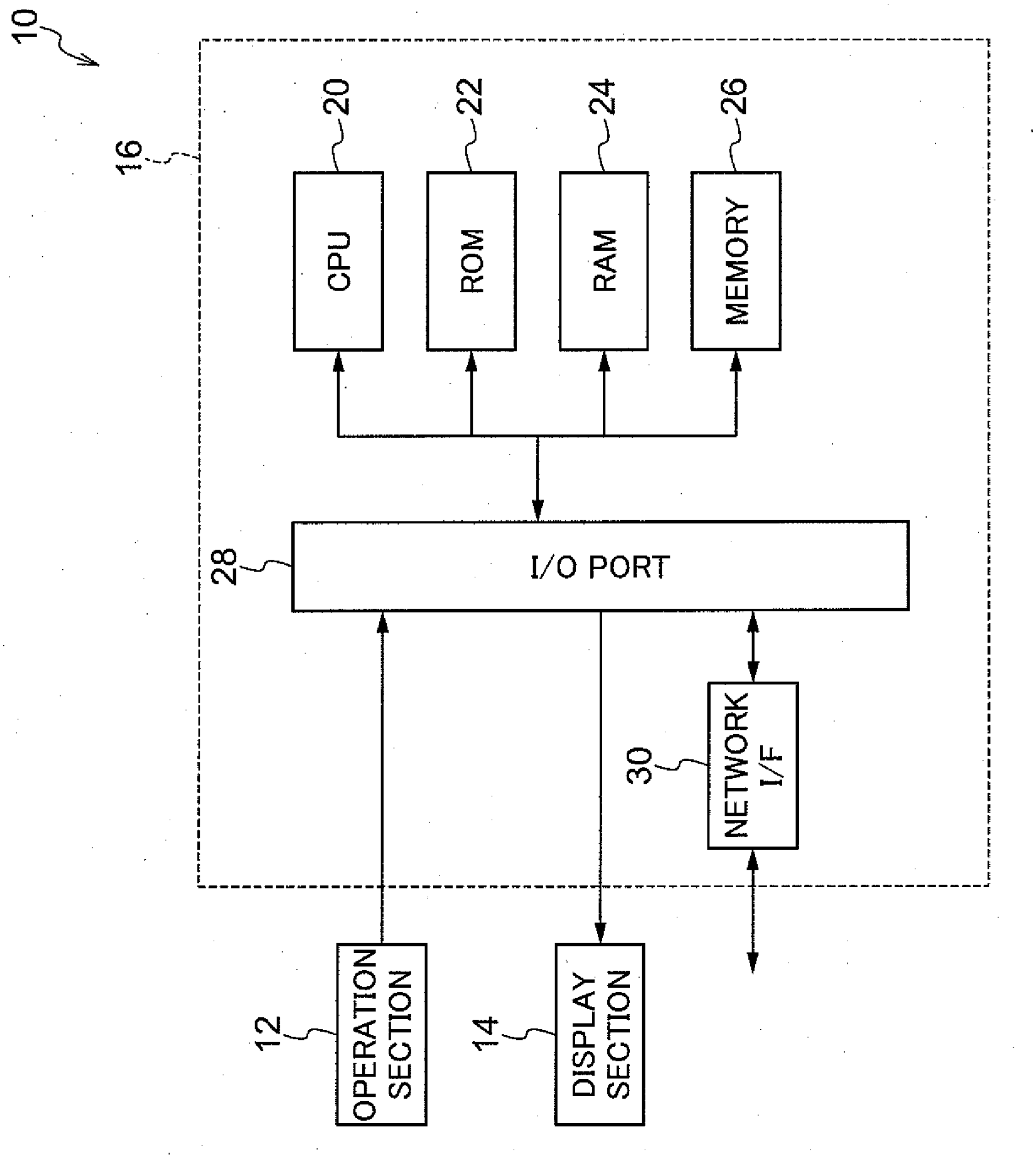
10
16
20
22
24
26
CPU
ROM
RAM
MEMORY
28
I/O PORT
30
NETWORK I/F
12
OPERATION SECTION
14
DISPLAY SECTION

FIG.2A

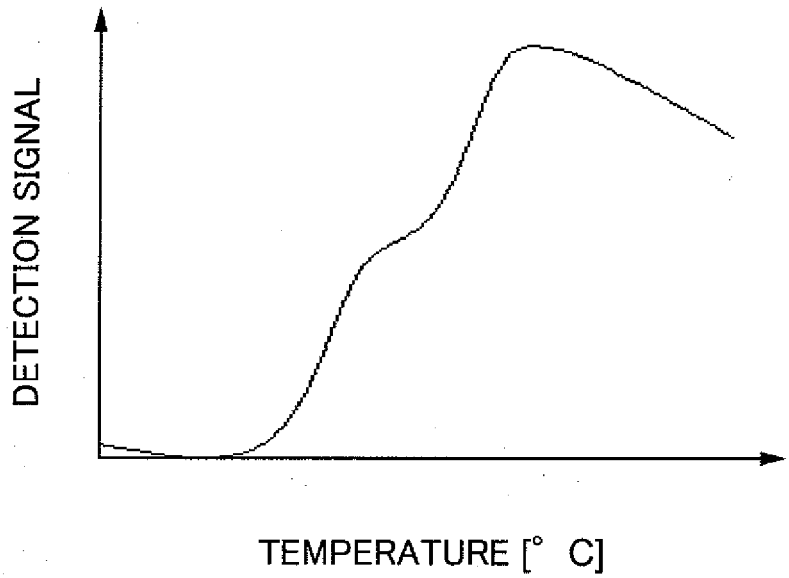
DETECTION SIGNAL
TEMPERATURE [° C]

FIG.2B

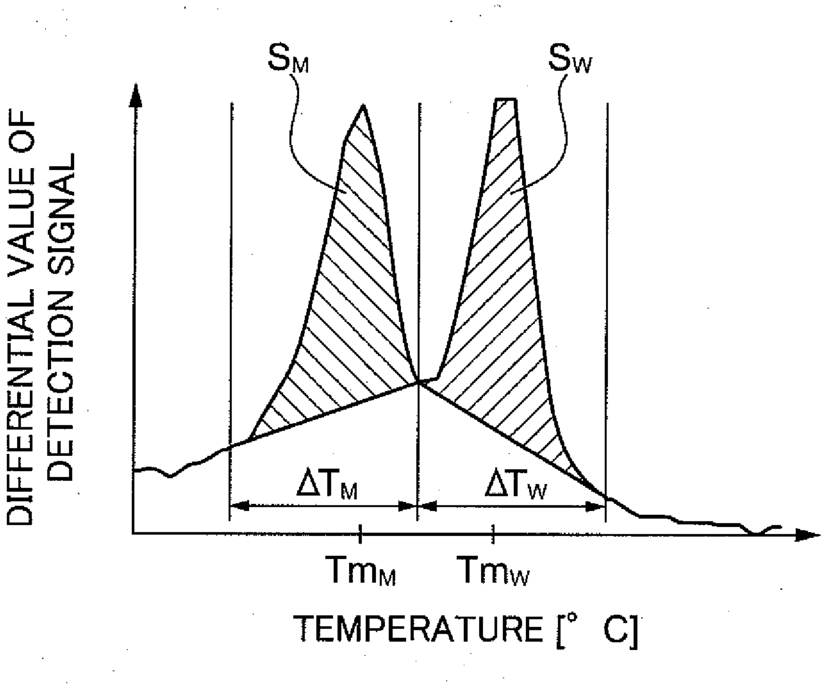
SM
SW
DIFFERENTIAL VALUE OF DETECTION SIGNAL
ΔTM
ΔTW
TmM
TmW
TEMPERATURE [° C]

FIG.3

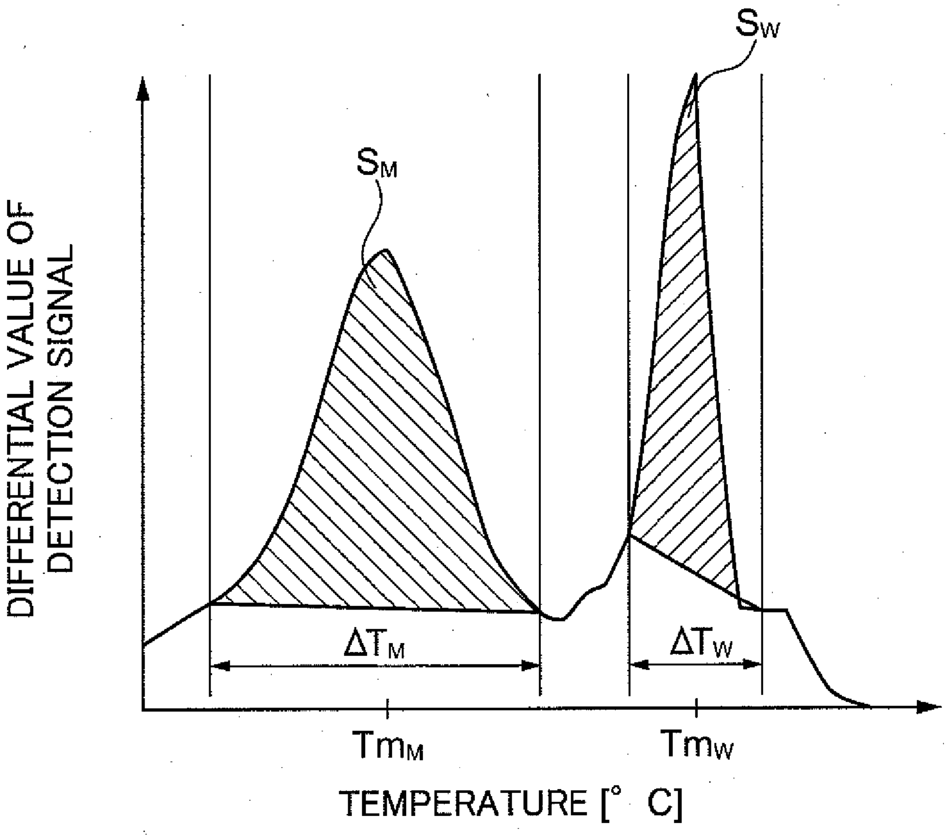
DIFFERENTIAL VALUE OF
DETECTION SIGNAL
SM
SW
ΔTM
ΔTW
TmM
TmW
TEMPERATURE [° C]

FIG.4

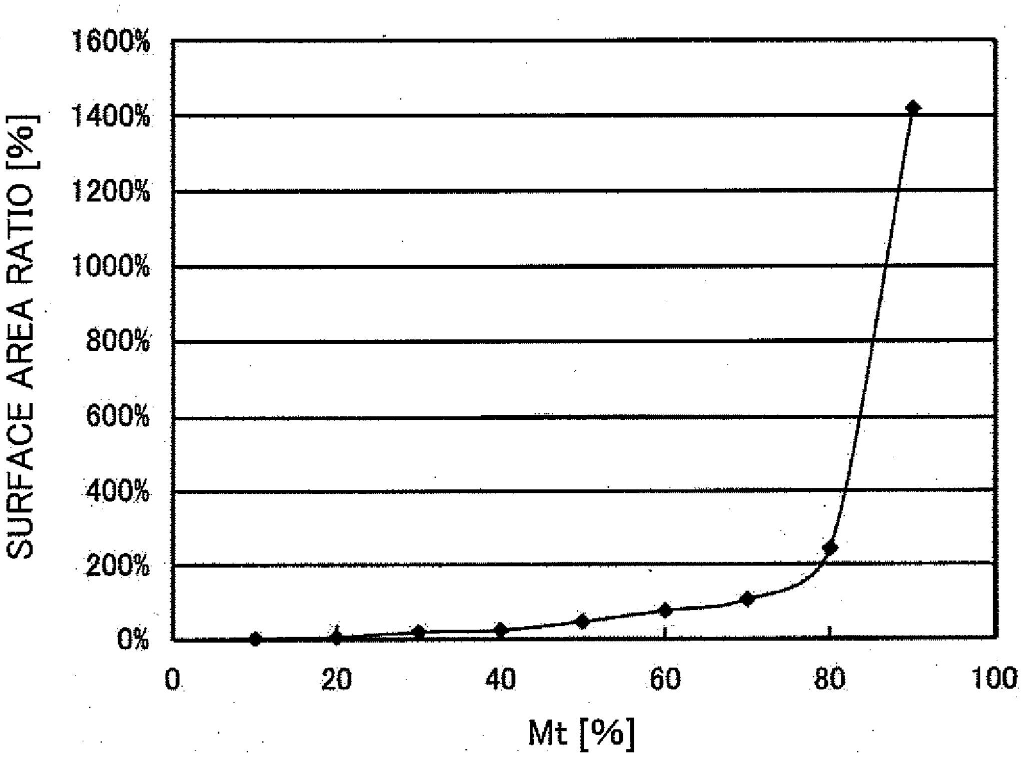
1600%
1400%
1200%
1000%
800%
600%
400%
200%
0%
SURFACE AREA RATIO [%]
0
20
40
60
80
100
Mt [%]

FIG.5

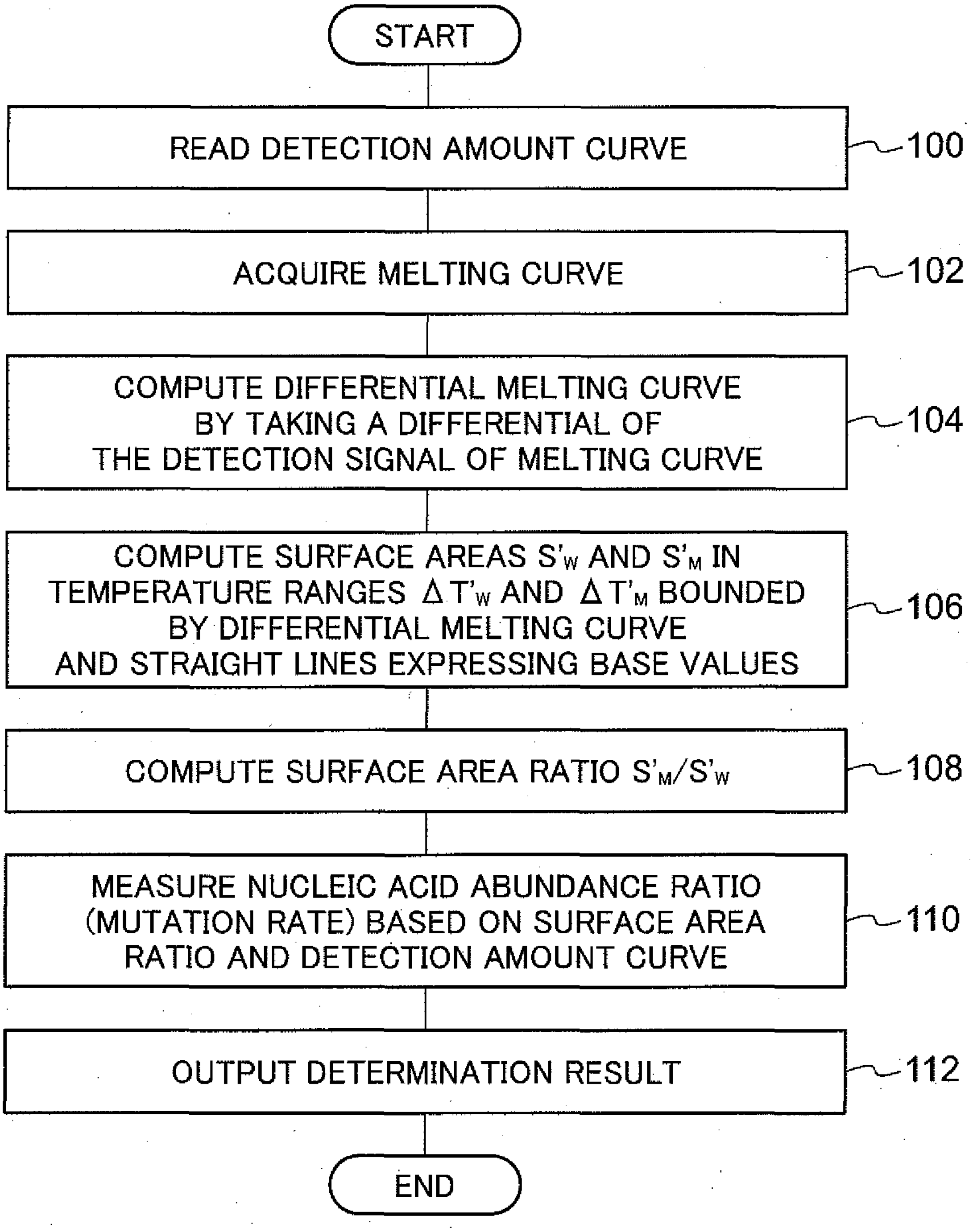
START
READ DETECTION AMOUNT CURVE
100
ACQUIRE MELTING CURVE
102
COMPUTE DIFFERENTIAL MELTING CURVE BY TAKING A DIFFERENTIAL OF THE DETECTION SIGNAL OF MELTING CURVE
104
COMPUTE SURFACE AREAS $S'_W$ AND $S'_M$ IN TEMPERATURE RANGES $\Delta T'_W$ AND $\Delta T'_M$ BOUNDED BY DIFFERENTIAL MELTING CURVE AND STRAIGHT LINES EXPRESSING BASE VALUES
106
COMPUTE SURFACE AREA RATIO $S'_M/S'_W$
108
MEASURE NUCLEIC ACID ABUNDANCE RATIO (MUTATION RATE) BASED ON SURFACE AREA RATIO AND DETECTION AMOUNT CURVE
110
OUTPUT DETERMINATION RESULT
112
END

FIG.6

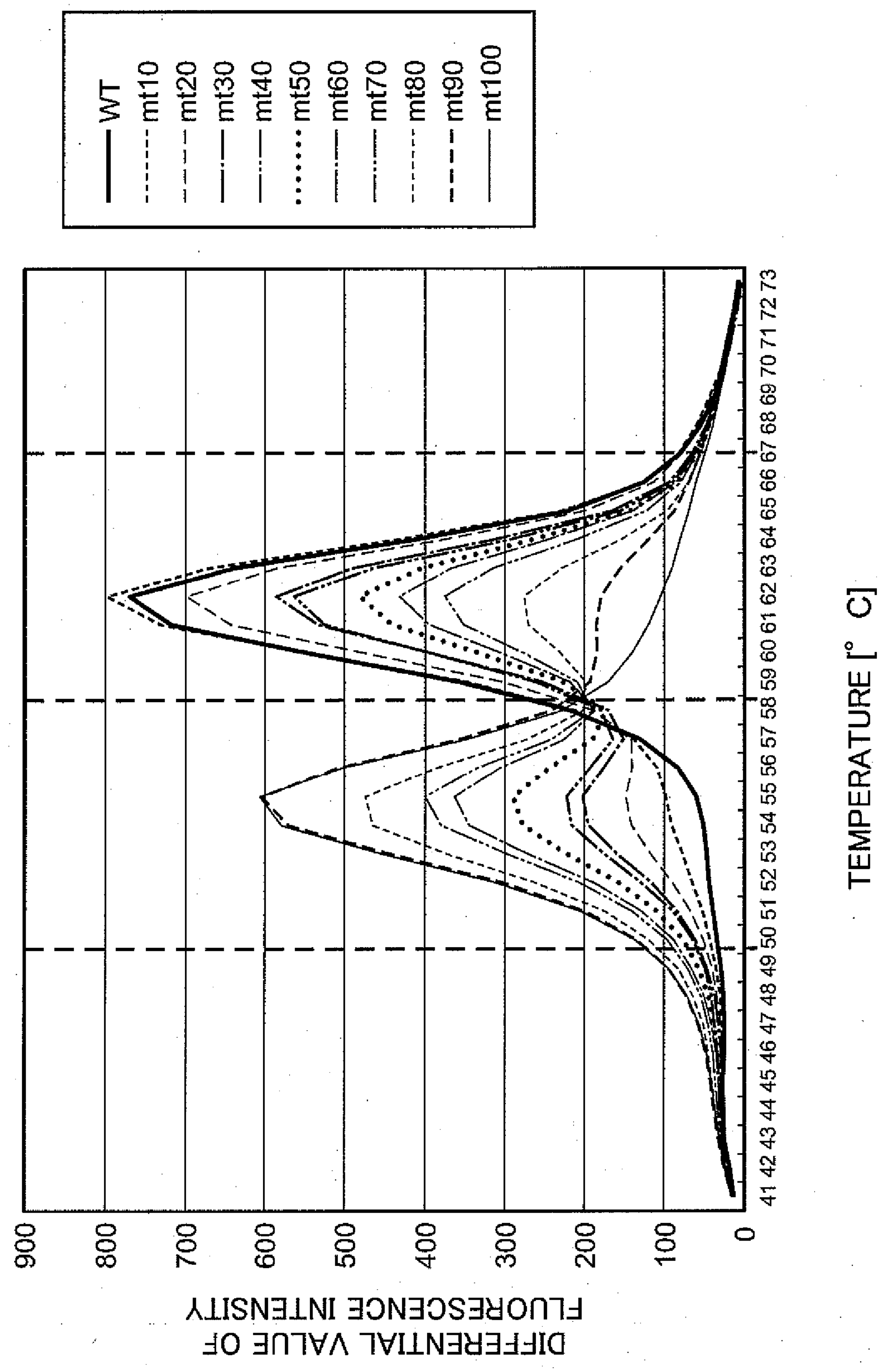
WT
mt10
mt20
mt30
mt40
mt50
mt60
mt70
mt80
mt90
mt100
DIFFERENTIAL VALUE OF FLUORESCENCE INTENSITY
900
800
700
600
500
400
300
200
100
0
41 42 43 44 45 46 47 48 49 50 51 52 53 54 55 56 57 58 59 60 61 62 63 64 65 66 67 68 69 70 71 72 73
TEMPERATURE [° C]

FIG.7

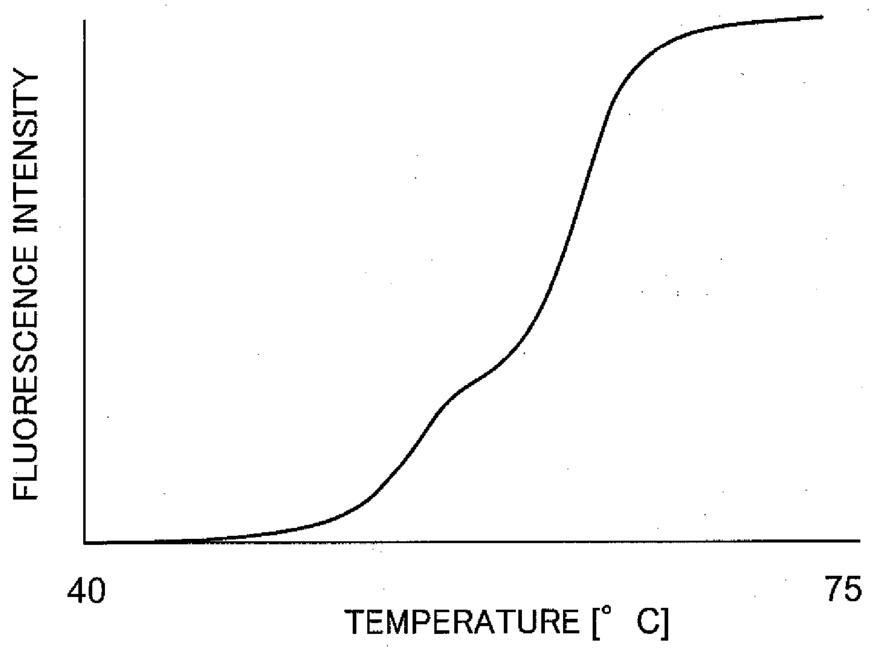
FLUORESCENCE INTENSITY
40
75
TEMPERATURE [° C]

FIG.8

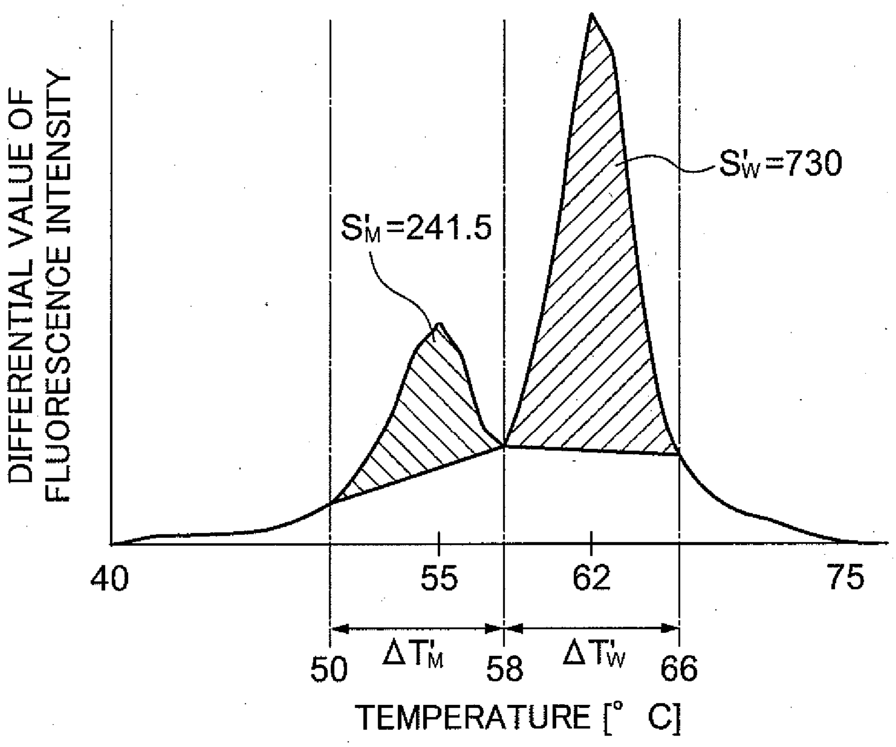
DIFFERENTIAL VALUE OF FLUORESCENCE INTENSITY
S'M=241.5
S'W=730
40
55
62
75
50
ΔT'M
58
ΔT'W
66
TEMPERATURE [° C]

FIG.9A

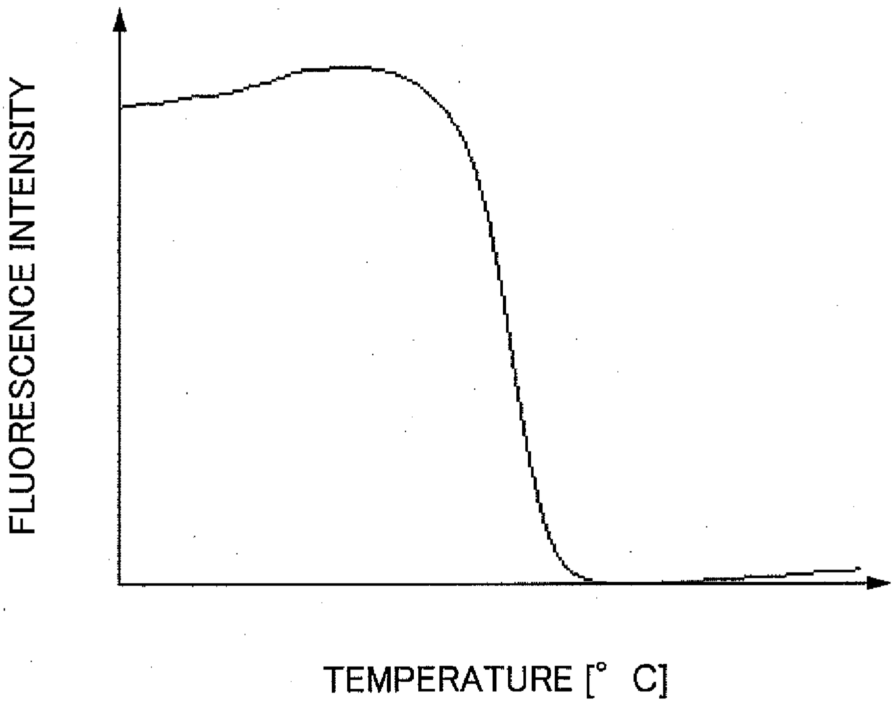
FLUORESCENCE INTENSITY
TEMPERATURE [° C]

FIG.9B

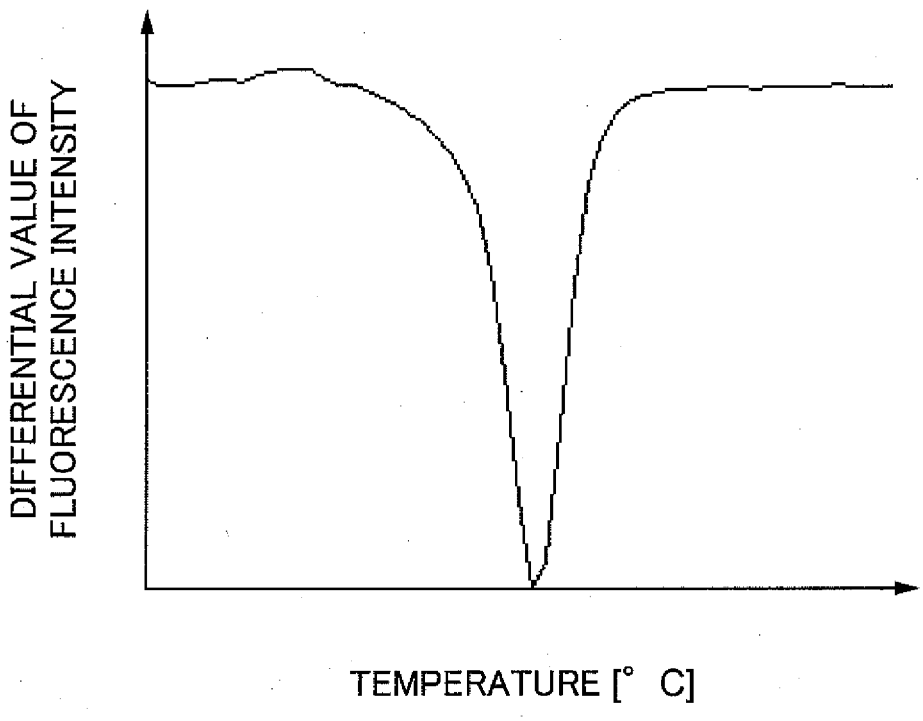
DIFFERENTIAL VALUE OF FLUORESCENCE INTENSITY
TEMPERATURE [° C]

FIG.10A

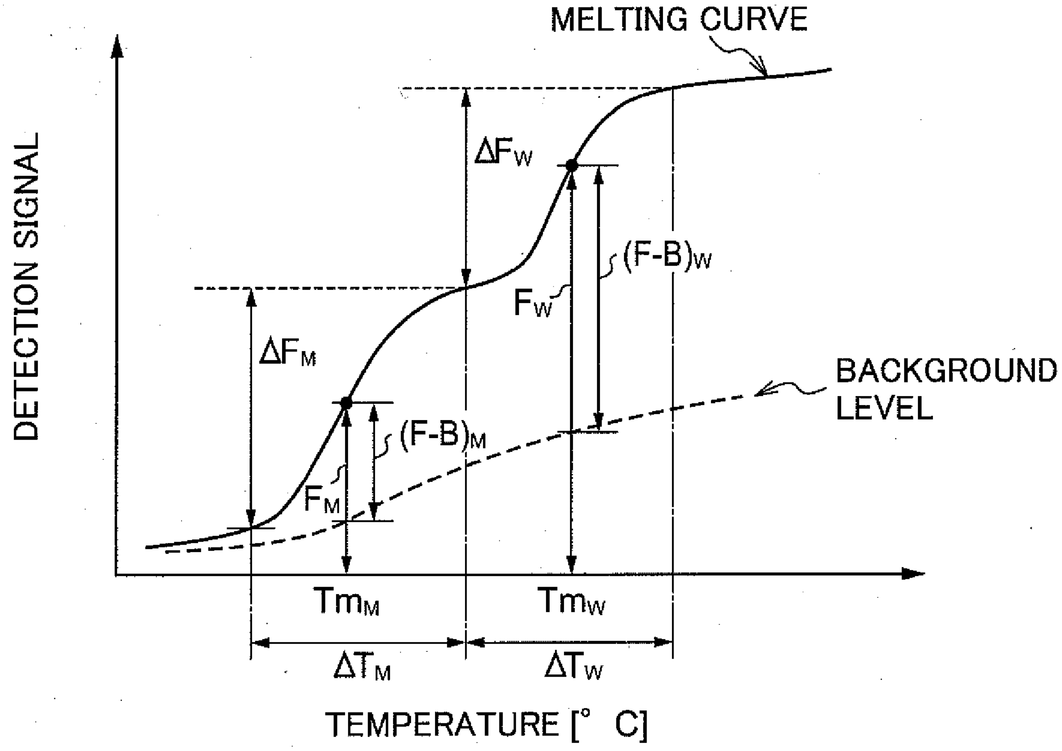


FIG.10B

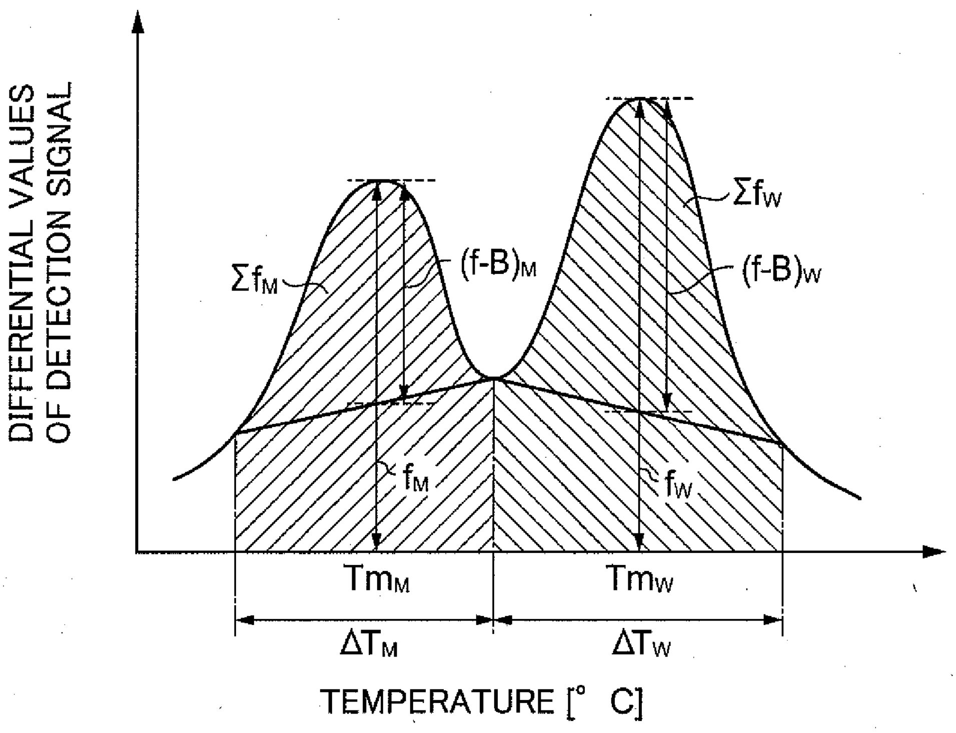

FIG.11

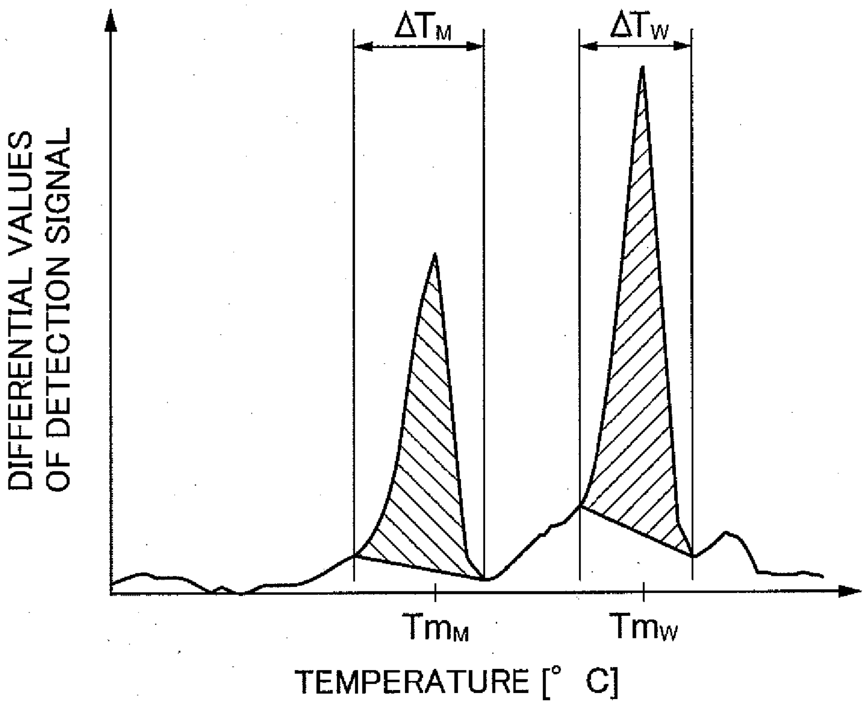
ΔTM
ΔTW
DIFFERENTIAL VALUES
OF DETECTION SIGNAL
TmM
TmW
TEMPERATURE [° C]

FIG.12

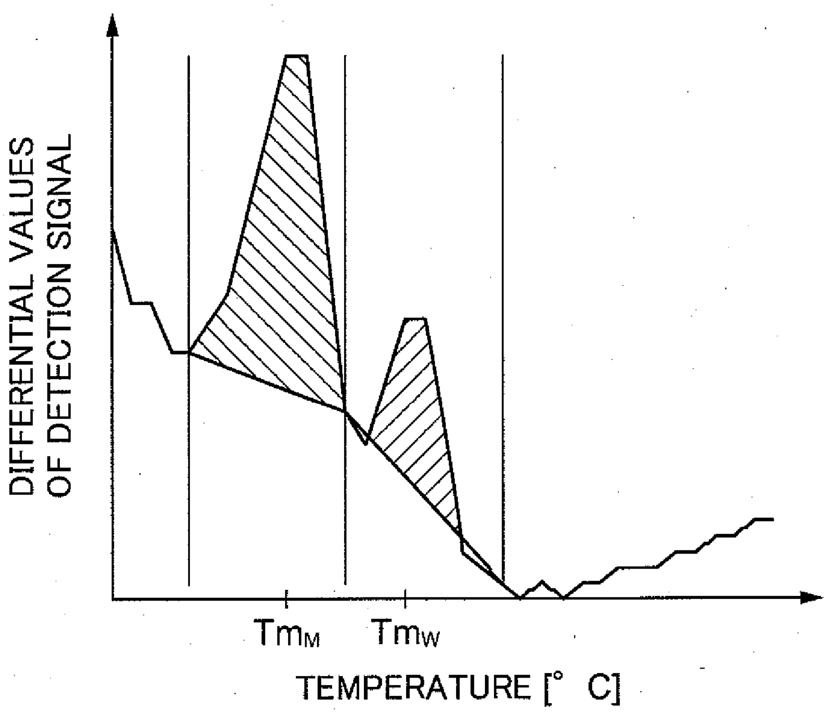
DIFFERENTIAL VALUES
OF DETECTION SIGNAL
TmM
TmW
TEMPERATURE [° C]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009081965 A
- US 2007224598 A
- US 2009155791 A
- JP 2007143420 A
- US 2007128608 A1
- JP 2010155032 A
- JP 2011148520 A


### Non-patent literature cited in the description


- SoFAR: Software for Fully Automatic Evaluation of Real-Time PCR Data. **WILHELM et al.** Biotechniques. Informa Healthcare, 2003, vol. 34:2, 324-332